# EUROPEAN PATENT SPECIFICATION

(11) **EP 0 519 370 B1**
(45) Date of publication and mention of the grant of the patent: **05.11.1997**
(21) Application number: 92110048.3
(22) Date of filing: 15.06.1992
(51) Int. Cl.: C07D 213/61, C09K 19/34, C07D 213/65, C07D 239/26, C07D 239/30, G02F 1/13

(54) **Heterocyclic derivatives**
Heterocyclische Derivate
Dérivés hétéro cyclique

(30) Priority: 21.06.1991 EP 91110261; 19.08.1991 EP 91113840
(43) Date of publication of application: 23.12.1992
(73) Proprietor: MERCK PATENT GmbH, D-64271 Darmstadt (DE)
(72) Inventor: Poetsch, Eike, Dr., W-6109 Mühltal 6 (DE); Meyer, Volker, W-6112 Gross-Zimmern 2 (DE); Hittich, Reinhard, Dr., W-6101 Modautal 1 (DE); Plach, Herbert, Dr., W-6100 Darmstadt (DE); Krause, Joachim, Dr., W-6110 Dieburg (DE); Kompter, Hans-Michael, Dr., W-6108 Weiterstadt (DE); Coates, David, Dr., Wimborne, Dorset BH21 3SW (GB); Smith, Graham, Poole, Dorset BH17 8AX (GB)

(56) References cited:
- EP-A- 0 169 139
- EP-A- 0 193 191
- EP-A- 0 332 024
- EP-A- 0 387 032
- CHEMICAL ABSTRACTS, vol. 115, no. 6, 12 August 1991, Columbus, Ohio, US; abstract no. 61016, page 795;
- CHEMICAL ABSTRACTS, vol. 110, no. 24, 12 June 1989, Columbus, Ohio, US; abstract no. 223255, page 721 ;
- MOLECULAR CRYSTALS AND LIQUID CRYSTALS (INC. NONLINEAR OPTICS ), vol. 206, 1991, READING, GB; pages 139 - 146, SHIN-ICHI SUGITA; 'Synthesis and Mesomorphic Properties of Ferroelectric Liquid Crystals Bearing 5-Phenylpyrimidine Rings'

## Description

The invention relates to heterocyclic derivatives of the formula I 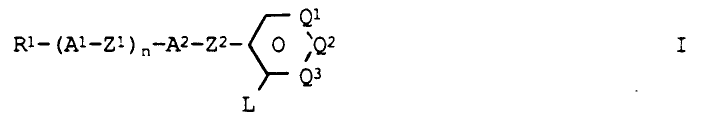 wherein
- R¹: denotes alkyl with up to 12 carbon atoms wherein one or two non-adjacent CH₂-groups may also be replaced by -O-, -O-CO-, -CO-O- and/or -CH=CH-,
- L: is H or F,
- A¹ and A²: in each case independently of one another are trans-1,4-cyclohexylene, wherein one or two non-adjacent CH₂-groups may also be replaced by -O- and/or -S-, or 1,4-phenylene which is unsubstituted or substituted by one or more halogen atoms and/or nitrile and/or CH₃ groups, and wherein one or more CH groups may also be replaced by N,
- Z¹ and Z²: in each case independently of one another are -CH₂CH₂-, -CO-O-, -O-CO-, -CH₂O-, -OCH₂-, -C≡C- or a single bond,
- n: is 0, 1 or 2,
one or two non-adjacent Q's denote N and the remaining bivalent groups Q¹, Q² and Q³ are C-halogen, and
in the case that Q¹ and Q³ are N Q² can also be C-O(CF₂)ₛ-Y or C-(CF₂)ₛ-Y, wherein
- Y: is H, F or Cl, and
- s: is 1 or 2,
with the exception of compounds of formula I where L is H, n is 0, Q¹ and Q² are N and Q³ is CCl and also a liquid crystalline medium being a mixture of at least two compounds, characterized in that at least one compound is a heterocyclic derivative according to formula I.

The invention was based on the object of discovering new stable liquid crystal or mesogenic compounds which are suitable as components of liquid crystalline media and, in particular have advantageous values of optical and dielectric anisotropy, in particular or the value of Δε/Δ⊥, combined with high nematogenity and favorable elastic properties.

Similar heterocyclic compounds with cyano as the terminal group instead of halogen atoms are, for example, described in EP 0194153, DD-A-145915, GB-A-2085877 and in EP-A-0186045.

JP 6 348 271 discloses 2-chloro-5-(4-n-decyloxyphenyl)-pyrimidine and EP 0 332 024 discloses 2-chloro-5-(4-n-octyl-2,3-difluorophenyl)pyrimidine as intermediate in liquid crystal synthesis.

In Molecular Crystals and Liquid Crystals, Vol. 206, 1991, pp. 139 - 146 2-chloro-5-phenylpyrimidines are mentioned as intermediates in the synthesis of liquid crystalline compounds. Furthermore, this document describes compounds possessing an alkyl or alkoxy side chain at p, p'-position of the 5-phenylpyrimidine core and the effect of structural changes on their mesomorphic properties. 2-Alkoxy substituted pyrimidines of this document exhibit no nematic state which is necessary for commercially interesting displays.

EP 0 169 139 describes 2-chloro-5-(4-methoxyphenyl)-pyrimidine as pharmaceutical.

It has been found, that the inventive compounds of the formula I give a significant smaller decrease of the holding ratio (S. Matsumoto et al., Liquid Crystals 5, 1320 (1983); G. Weber et al., loc. cit. p. 1381 (1989)) than corresponding compounds with a terminal cyano group exposed to UV radiation and higher temperatures.

It has now been found that heterocyclic derivatives of the formula I are highly suitable as components of liquid crystalline media.

It is also possible to obtain stable liquid crystal phases with a broad nematic mesophase range including a good deep temperature behaviour a low threshold voltage and a comparatively low viscosity with the aid of these compounds.

Depending on the choice of the substituents, the compounds of the formula I can be used as the base materials from which liquid crystal media are predominantly composed; however, it is also possible for compounds of the formula I to be added to liquid crystal base materials of other classes of compounds, for example in order to influence the dielectric and/or optical anisotropy and/or the elastic properties and/or the viscosity and/or the nematic mesophase range of such a dielectric.

The compounds of the formula I are colourless in the pure state and are liquid crystalline in a temperature range which is favourable placed for electrooptical use. They are very stable towards chemicals, heat and light.

The invention thus relates to the heterocyclic derivatives of the formula I, to liquid crystalline media with at least two liquid crystalline compounds, wherein at least one component is a compound of the formula I and to liquid crystal display devices containing such media.

Above and below R¹, L, A¹, A², Z¹, Z², n, Q¹, Q², and Q³ have the meaning indicated, unless something else is specifically stated.

For the sake of simplicity in the following, Phe is a 1,4-phenylene group which may be substituted by one or more halogen atoms and/or CN and/or CH₃ groups, Cyc is a trans-1,4-cyclohexylene group, Dio is a 1,3-dioxane-2,5-diyl group, Dit is a 1,3-dithian-2,5-diyl group, Pyd is a pyridine-2,5-diyl group and Pyr is a pyrimidine-2,5-diyl group. Het denotes the group 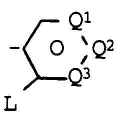 and comprises the structural elements

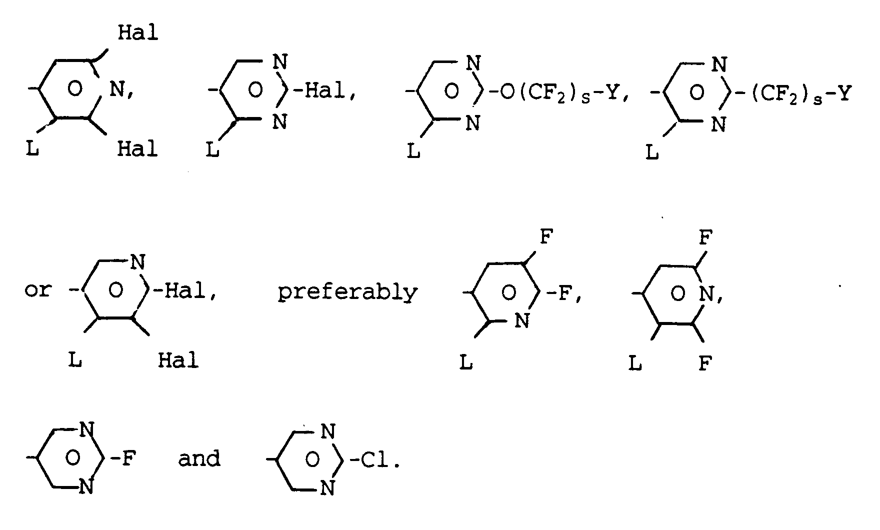

The compounds of the formula I accordingly also include compounds of the partial formulae Ia to Ib (having two rings), Ic to If (having three rings) and Ig to In (having four rings):

R¹-A²-Het Ia

R¹-A²-Z²-Het Ib

R¹-A¹-A²-Het Ic

R¹-A¹-A²-Z²-Het Id

R¹-A¹-Z¹-A²-Het Ie

R¹-A¹-Z¹-A²-Z²-Het If

R¹-A¹-A¹-A²-Het Ig

R¹-A¹-Z¹-A¹-A²-Het Ih

R¹-A¹-A¹-Z¹-A²-Het Ii

R¹-A¹-A¹-A²-Z²-Het Ij

R¹-A¹-Z¹-A¹-Z¹-A²-Het Ik

R¹-A¹-Z¹-A¹-A²-Z²-Het Il

R¹-A¹-A¹-Z¹-A²-Z²-Het Im

R¹-A¹-Z¹-A¹-Z¹-A²-Z²-Het In

Among these, those of the formulae Ia, Ib, Ic, Id, Ie, Ih, Ii, Ik and Il are particularly preferred.

The preferred compounds of the partial formulae Ia include those of the partial formulae Iaa to Iad:

R¹-Phe-Het Iaa

R¹-Cyc-Het Iab

R¹-Pyd-Het Iac

R¹-Pyr-Het Iad

Among these, those of the formulae Iaa and Iab are particularly preferred.

The preferred compounds of the partial formula Ib include those of the partial formulae Iba to Ibj:

R¹-Phe-C≡C-Het Iba

R¹-Cyc-C≡C-Het Ibb

R¹-Phe-CO-O-Het Ibc

R¹-Cyc-CO-O-Het Ibd

R¹-Phe-CH₂CH₂-Het Ibe

R¹-Cyc-CH₂CH₂-Het Ibf

R¹-Phe-CH₂O-Het Ibg

R¹-Cyc-CH₂O-Het Ibh

R¹-Pyd-Z²-Het Ibi

R¹-Pyr-Z²-Het Ibj

Among these, those of the formulae Iba, Ibb, Ibc, Ibd, Ibe and Ibf are particularly preferred.

The preferred compounds of the partial formula Ic include those of the partial formulae Ica to Ici:

R¹-Cyc-Cyc-Het Ica

R¹-Phe-Phe-Het Icb

R¹-Dio-Phe-Het Icc

R¹-Dit-Phe-Het Icd

R¹-Cyc-Phe-Het Ice

R¹-Phe-Pyd-Het Icf

R¹-Phe-Pyr-Het Icg

R¹-Phe-Cyc-Het Ich

R¹-Cyc-Phe-Het Ici

Among these, those of the partial formulae Ica, Icb, Ice, Icf and Ici are particularly preferred.

The preferred compounds of the partial formula Id include those of the partial formulae Ida to Idm:

R¹-Cyc-Cyc-C≡C-Het Ida

R¹-Phe-Phe-C≡C-Het Idb

R¹-Cyc-Phe-C≡C-Het Idc

R¹-Cyc-Phe-CH₂CH₂-Het Idd

R¹-Cyc-Cyc-CH₂CH₂-Het Ide

R¹-Phe-Phe-CH₂CH₂-Het Idf

R¹-Cyc-Cyc-COO-Het Idg

R¹-Phe-Cyc-COO-Het Idh

R¹-Phe-Phe-COO-Het Idi

R¹-Cyc-Phe-COO-Het Idj

R¹-Phe-Phe-CH₂O-Het Idk

R¹-Cyc-Phe-CH₂O-Het Idl

R¹-Cyc-Cyc-CH₂O-Het Idm

Among these, those of the formulae Ida, Idb, Idc, Idd, Ide, Idg and Idj are particularly preferred.

The preferred compounds of the partial formula Ie include those of the partial formulae Iea to Iem:

R¹-Cyc-CH₂CH₂-Phe-Het Iea

R¹-Phe-CH₂CH₂-Phe-Het Ieb

R¹-Cyc-C≡C-Phe-Het Iec

R¹-Phe-C≡C-Phe-Het Ied

R¹-Phe-CH₂O-Phe-Het Iee

R¹-Cyc-COO-Phe-Het Ief

R¹-Cyc-COO-Cyc-Het Ieg

R¹-A¹-OCO-Phe-Het Ieh

R¹-A¹-OCH₂-Phe-Het Iei

R¹-A¹-Z¹-Pyd-Het Iej

R¹-A¹-Z¹-Pyr-Het Iek

R¹-Cyc-C≡C-Cyc-Het Iel

R¹-Cyc-CH₂CH₂-Cyc-Het Iem

Among these, those of partial formulae Iea, Ieb, Iec, Ied, Ieg and Iem are particularly preferred.

The preferred compounds of the partial formula If include those of the partial formulae Ifa to Ifh:

R¹-A¹-Z¹-Phe-C≡C-Het Ifa

R¹-A¹-CH₂CH₂-Phe-CH₂CH₂-Het Ifb

R¹-A¹-Z¹-Phe-CH₂CH₂-Het Ifc

R¹-A¹-Z¹-Cyc-COO-Het Ifd

R¹-Cyc-Z¹-Cyc-C≡C-Het Ife

R¹-Cyc-Z¹-Cyc-CH₂CH₂-Het Iff

R¹-A¹-COO-Phe-Z²-Het Ifg

R¹-A¹-CH₂O-Phe-Z²-Het Ifh

The preferred compounds of the formulae Ig to In include those of the partial formulae Iga to Igo:

R¹-A¹-Cyc-Cyc-Het Iga

R¹-Cyc-Cyc-Phe-Het Igb

R¹-Phe-Phe-Cyc-Het Igc

R¹-A¹-CH₂CH₂-A¹-Cyc-Het Igd

R¹-Cyc-COO-A¹-Phe-Het Ige

R¹-A¹-A¹-CH₂CH₂-Phe-Het Igf

R¹-Cyc-Cyc-Z¹-A²-Het Igg

R¹-A¹-Phe-Phe-CC-Het Igh

R¹-A¹-Cyc-Phe-CC-Het Igi

R¹-A¹-A¹-A²-CH₂CH₂-Het Igj

R¹-A¹-A¹-A²-COO-Het Igk

R¹-Cyc-COO-A¹-Z¹-Phe-Het Igl

R¹-Phe-C≡C-A¹-Z¹-Phe-Het Igm

R¹-A¹-CH₂CH₂-Cyc-Cyc-CH₂CH₂-Het Ign

R¹-A¹-Z¹-Phe-Phe-Z²-Het Igo

In the compounds of the formulae above and below. L is H or F.

In the compounds of the formulae above and below R¹ is preferably alkyl, alkoxy, oxaalkyl, alkanoyloxy or alkenyl and can exhibit a straight-chain or branched structure.

Alkyl or alkoxy preferably are straight-chain and have 2, 3, 4, 5, 6 or 7 C atoms. Accordingly they are preferably ethyl, propyl, butyl, pentyl, hexyl, heptyl, ethoxy, propoxy, butoxy, pentoxy, hexoxy, or heptoxy, also methyl, octyl, nonyl, decyl, undecyl, dodecyl, methoxy, octoxy, nonoxy, decoxy or undecoxy.

Oxaalkyl is preferably straight-chain 2-oxapropyl (= methoxymethyl), 2-(= ethoxymethyl) or 3-oxybutyl (= 2-methoxyethyl), 2-, 3- or 4-oxapentyl, 2-, 3-, 4- or 5-oxahexyl, 2-, 3-, 4- 5- or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-, 7-, 8-, or 9-oxadecyl.

Alkenyl is preferably straight-chain and has 2 to 10 C atoms. It is accordingly, in particular, vinyl, prop-1- or prop-2-e-nyl, but-1-, -2- or -3-enyl, pent-1-, -2-, -3- or -4-enyl, hex-1-, -2-, -3-, -4- or -5-enyl, hept-1-, -2-, -3-, -4-, -5- or -6-enyl, oct-1-, -2-, -3-, -4-, -5- -6- or -7-enyl, non-1-, -2-, -3-, -4-, -5-, -6-, -7- or -8-enyl or dec-1-, -2-, -3-, -4-, -5-, -6-, -7-, -8- or -9-enyl.

Compounds of the formula I containing a branched terminal group can occasionally be of importance because of an improved solubility in the customary liquid crystal base materials, but in particular as chiral doping substances if they are optically active.

Branched groups of this type as a rule contain not more than one chain branching. Preferred branched radicals are isopropyl, 2-butyl (= 1-methylpropyl),isobutyl (= 2-methylpropyl, 2-methylbutyl, isopentyl, (= 3-methylbutyl), 2-methylpentyl, 2-ethylhexyl, 2-propylpentyl, 2-octyl, isopropoxy, 2-methyl-propoxy, 2-methylbutoxy, 3-methylbutoxy, 2-methylpentoxy, 3-methylpentoxy, 2-ethylhexoxy, 2-methylhexoxy, 1-methylhexoxy, 1-methylheptoxy (= 2-octyloxy), 2-oxa-3-methylbutyl, 3-oxa-4-methylpentyl, 4-methylhexyl, 2-nonyl, 2-decyl, 2-dodecyl, 6-methyloctoxy, 6-methyloctanoyloxy, 4-methylheptyloxycarbonyl, 2-methylbutyryloxy, 3-methylvaleryloxy, 4-methylhexanoyloxy, 2-methyl-3-oxapentyl and 2-methyl-3-oxahexyl.

In the case of compounds with a branched terminal group R¹, formula I includes both the optical antipodes and racemates as well as mixtures thereof.

n is preferably 0 or 1. If n is 2, the two groups A¹ and Z¹ may be identical or different from one another.

Z¹ and Z² are preferably a single bond, a -CH₂CH₂- or a -CO-O-group, also -O-CO-, -CH₂O- or -OCH₂-. Preferably all groups Z¹ and Z² present in the molecule are single bonds or alternatively one of these groups is -CH₂CH₂-.

A¹ and A² are preferably unsubstituted 1,4-phenylene or trans 1,4-cyclohexylene. Compounds wherein A¹ and/or A² are substituted 1,4-phenylene are also preferred. They are then preferably mono- or disubstituted by fluorine and the 1,4-phenylene group has preferably the following structures: 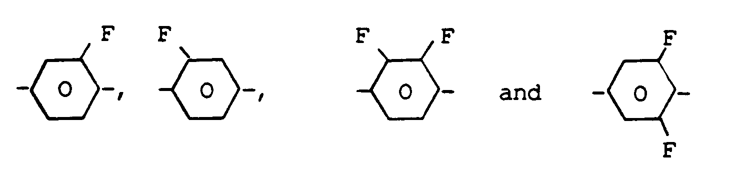 A¹ and A² also preferably have the meaning of Pyd or Pyr.

The following group of compounds of the formula I is particularly preferred:

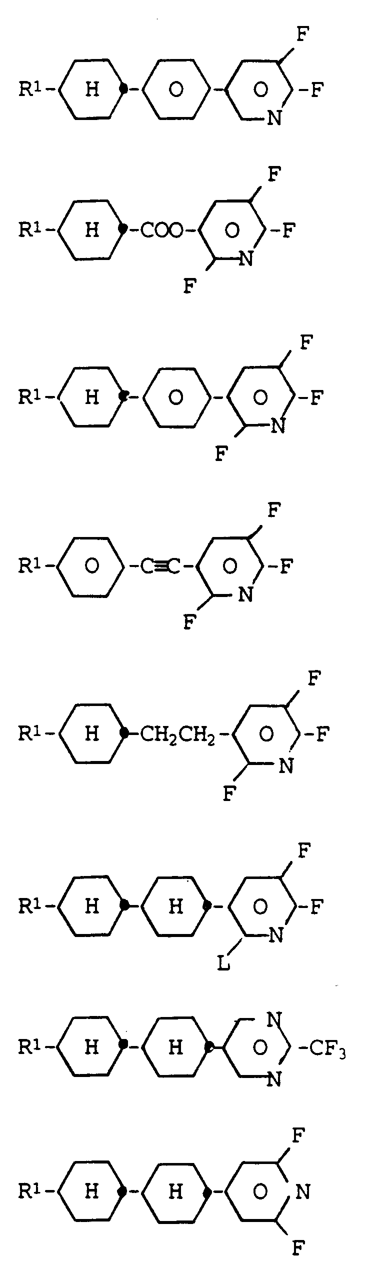

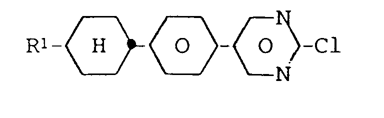

The compounds of the formula I are prepared by methods which are known per se, such as are described in the literature (for example in the standard works, such as Houben-Weyl, Methoden der Organischen Chemie [Methods of Organic Chemistry], Georg Thieme Verlag, Stuttgart), and in particular under reaction conditions which are known and suitable for the reactions mentioned. Variants which are known per se and are not mentioned in more detail here can also be used in this connection.

If desired, the starting materials can also be formed in situ, such that they are not isolated from the reaction mixture but are immediately reacted further to give the compounds of the formula I.

The most important starting materials for most of the synthesis routes are the following substances:

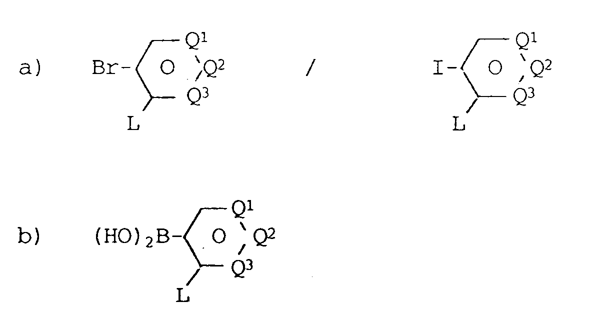

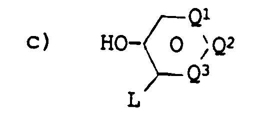

These key intermediates are preferably made by the following routes (LDA = lithiumdiisopropylamide).

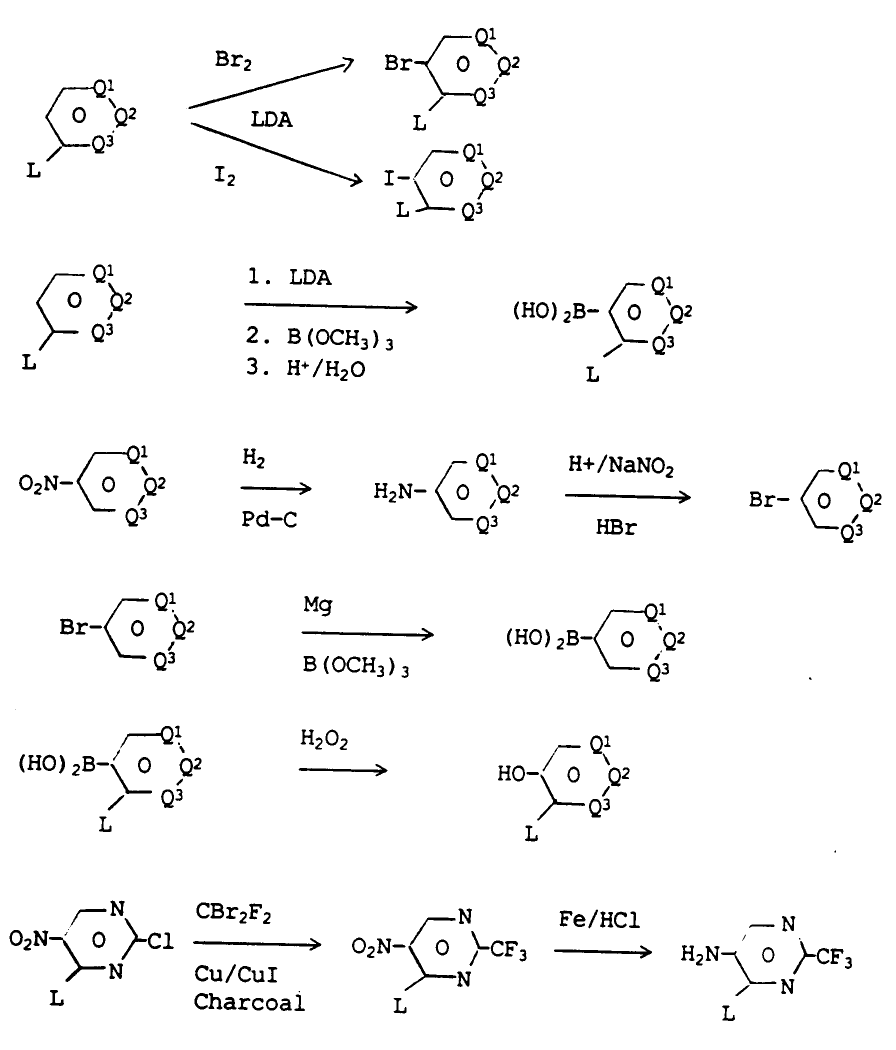

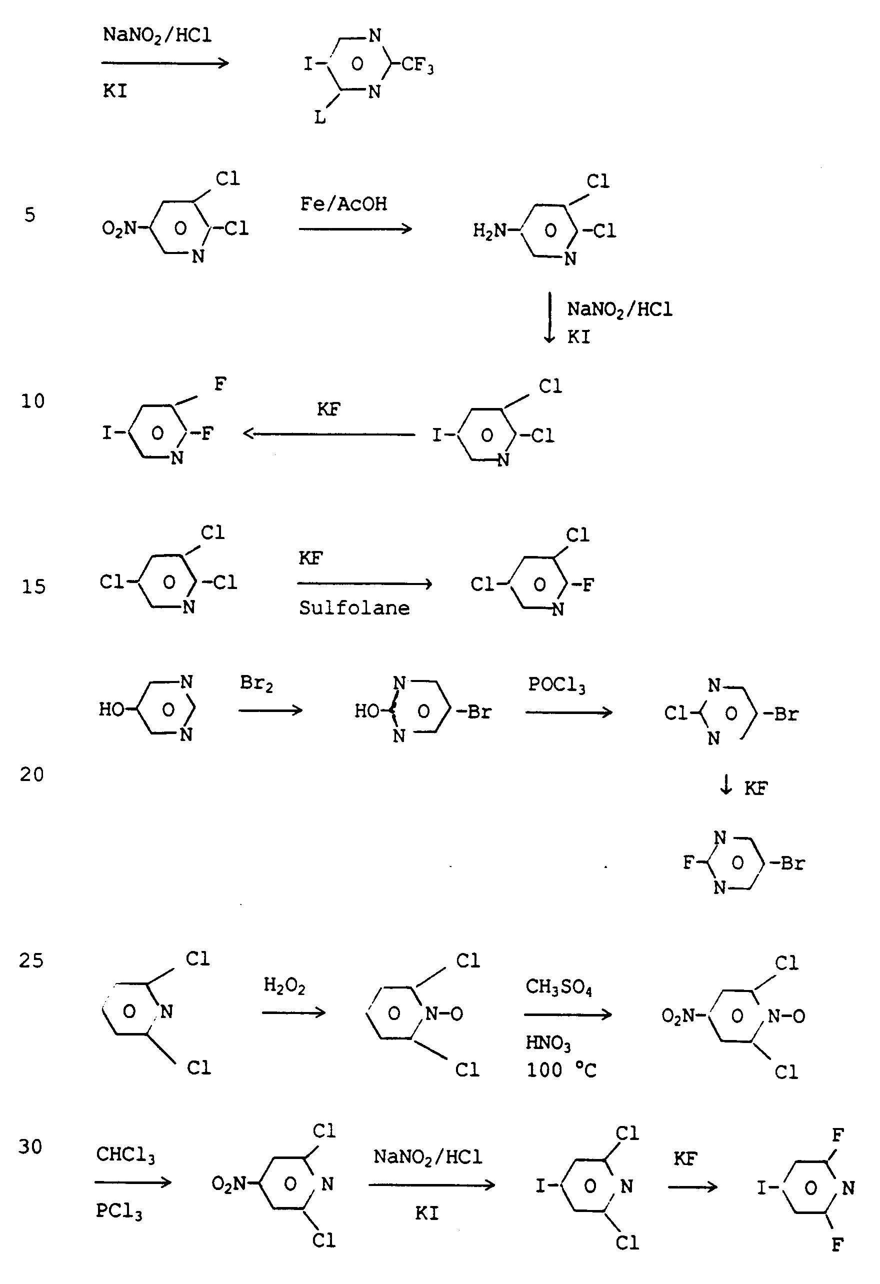

The compounds of the formula I thus can be prepared preferably by the following coupling reactions which are known per se:

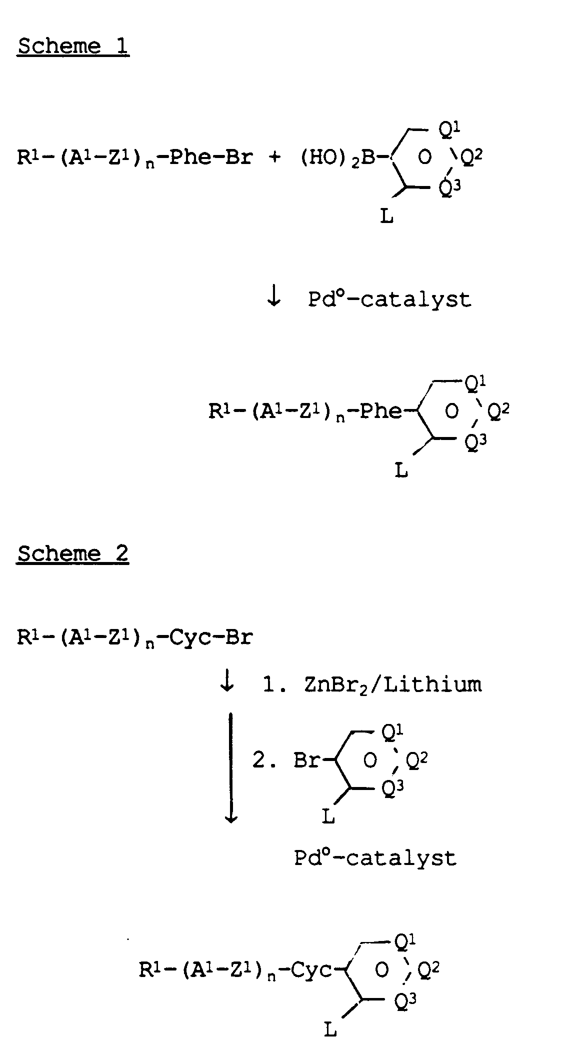

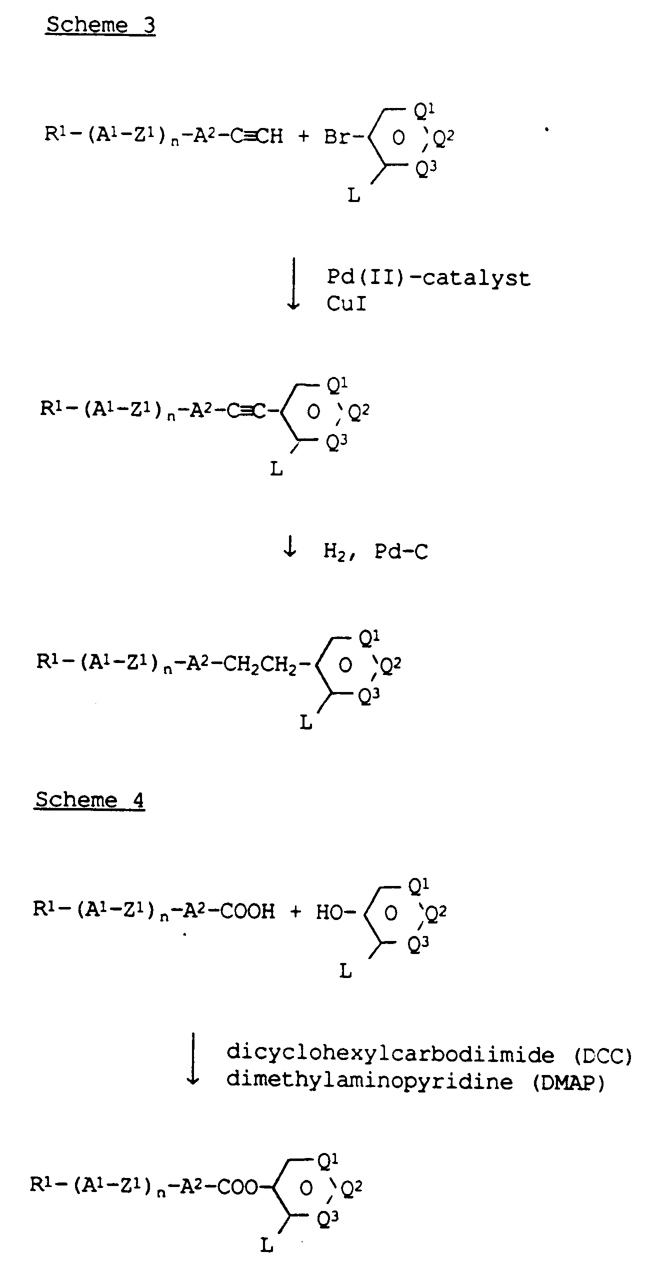

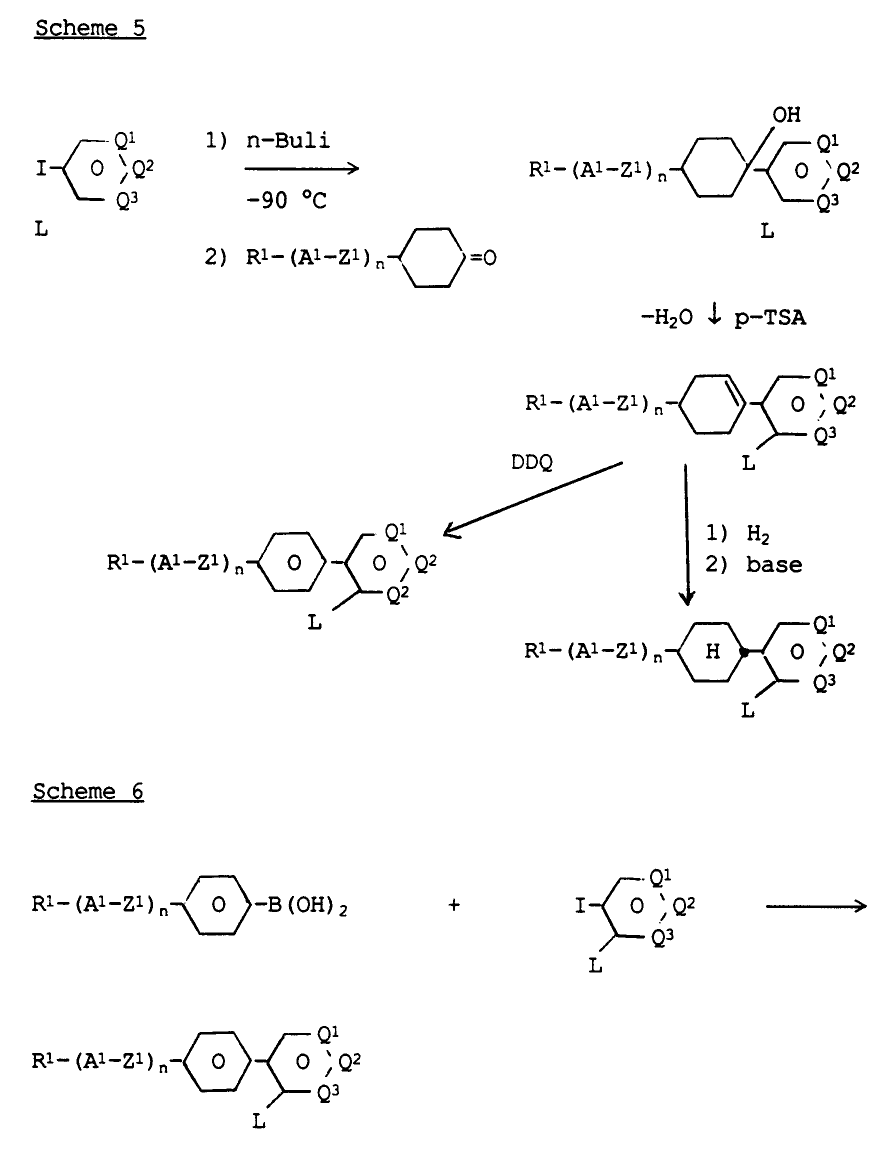

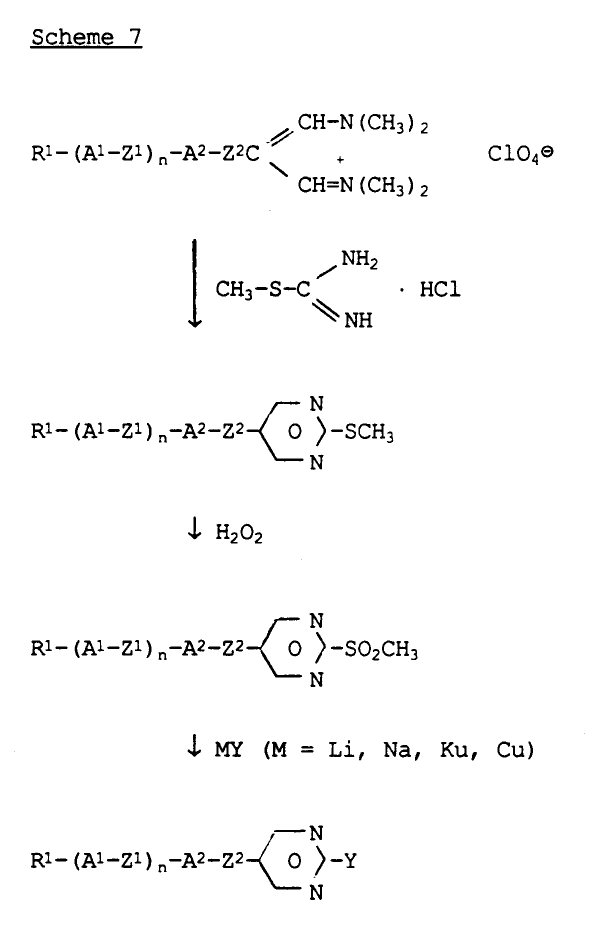

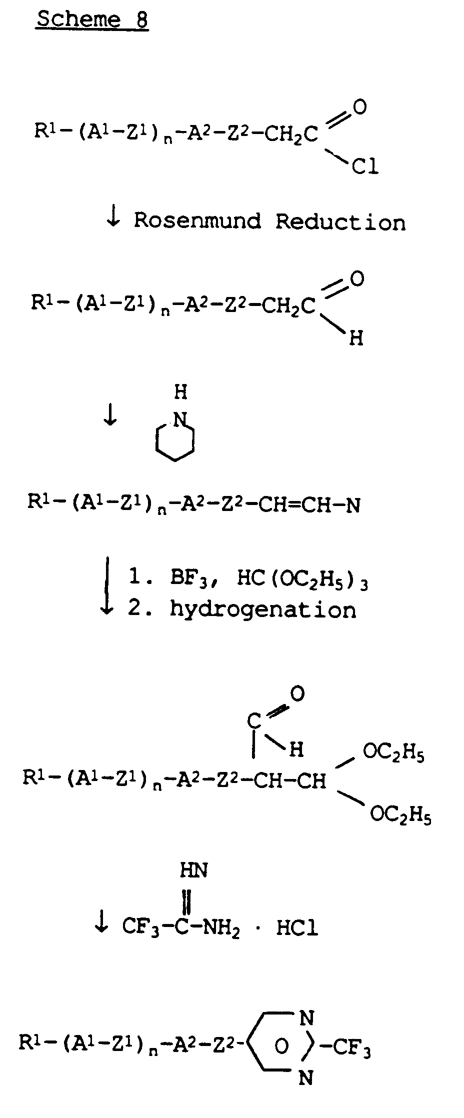

The reaction conditions are known to the skilled worker or described in the literature already mentioned. Other routes are apparent to the skilled worker.

In addition to one or more compounds of the formula I the liquid crystal media according to the invention preferably contain 2-40 components and in particular 4-30 components. Liquid crystal media being composed of one or more compounds of formula I and 7-25 other components are especially preferred.

These additional components are preferably chosen from the nematic or nematogenic (monotropic or isotropic) substances; in particular from the classes of azoxybenzenes, benzylideneanilines, biphenyls, terphenyls, phenyl or cyclohexyl benzoates, phenyl or cyclohexyl cyclohexanecarboxylates, phenyl or cyclohexyl cyclohexylbenzoates, phenyl or cyclohexyl cyclohexylcyclohexanecarboxylates, cyclohexylphenylbenzoates, cyclohexylphenyl cyclohexanecarboxylates, cyclohexylphenyl cyclohexylcyclohexanecarboxylates, phenylcyclohexanes, cyclohexylbiphenyls, phenylcyclohexylcyclohexanes, cyclohexylcyclohexanes, cyclohexylcyclohexenes, cyclohexylcyclohexylcyclohexene, 1,4-bis-cyclohexylbenzenes, 4,4'-bis-cyclohexylbiphenyls, phenyl- or cyclohexylpyrimidines, phenyl- or cyclohexylpyridines, phenyl- or cyclohexyldioxanes, phenyl- or cyclohexyl-1,3-dithianes, 1,2-diphenylethanes, 1,2-dicyclohexylethanes, 1-phenyl-2-cyclohexylethanes, 1-cyclohexyl-2-(4-phenyl-cyclohexyl)-ethanes, 1-cyclohexyl-2-biphenylethanes, 1-phenyl-2-cyclohexyl-phenylethanes, optionally halogenated stilbenes, benzyl phenyl ethers, tolanes and substituted cinnamic acids.

The 1,4-phenylene groups of these compounds may be fluorinated.

The most important compounds which are possible constituents of liquid crystal media according to the invention can be characterized by the formalae 1, 2, 3, 4 and 5:

R'-L-U-R'' 1

R'-L-COO-U-R'' 2

R'-L-OOC-U-R'' 3

R'-L-CH₂CH₂-U-R'' 4

R'-L-C≡C-U-R'' 5

In the formulae 1, 2, 3, 4 and 5 L and U may be equal or different from each other. L and U independently from each other denote a bivalent residue selected from the group consisting of -Phe-, -Cyc-, -Phe-Phe-, -Phe-Cyc-, -Cyc-Cyc-, -Pyr-, -Dio-, -G-Phe-, -G-Cyc- and their mirror images; in this compilation of residues Phe denotes unsubstituted or fluorinated 1,4-phenylene, Cyc trans- 1,4-cyclohexylene or 1,4-cyclohexenylene, Pyr pyrimidine-2,5-diyl or pyridine-2,5-diyl, Dio 1,3-dioxane-2,5-diyl and G 2-(trans-1,4-cyclohexyl)-ethyl, pyrimidine-2,5-diyl, pyridine-2,5-diyl or 1,3-dioxane-2,5-diyl.

One of the residues L and U is preferably Cyc, Phe or Pyr. U preferably denotes Cyc, Phe or Phe-Cyc. The liquid crystal media according to the invention preferably contain one or more components selected from the compounds of formulae 1, 2, 3, 4 and 5 with L and U meaning Cyc, Phe and Pyr, said liquid crystal media further containing at the same time one ore more components selected from the compounds of formulae 1, 2, 3, 4 and 5 with one of the residues L and U denoting Cyc, Phe and Pyr and the other residue being selected from the group consisting of -Phe-Phe-, -Phe-Cyc-, -Cyc-Cyc-, -G-Cyc-, said liquid crystal media containing in addition to this optionally one or more components selected from the compounds of formulae 1, 2, 3, 4 and 5 with L and U being selected from the group consisting of -Phe-Cyc-, -Cyc-Cyc-, -G-Phe- and -G-Cyc.

In a preferred subgroup of the compounds of formulae 1, 2, 3, 4 and 5 (subgroup 1) R' and R'' are independently from each other alkyl, alkenyl, alkoxy, alkenoxy with up to 8 carbon atoms. R' and R'' differ from one another in most of these compounds, one of the residues usually being alkyl or alkenyl. In another preferred subgroup of the compounds of formulae 1, 2, 3, 4 and 5 (subgroup 2) R'' denotes -CN, -CF₃, -F, -Cl or -NCS while R' has the meaning indicated in subgroup 1 and is preferably alkyl or alkenyl. Other variants of the envisaged substituents in the compounds of formulae 1, 2, 3, 4 and 5 are also customary. Many such substances are commercially available. All these substances are obtainable by methods which are known from the literature or by analogous methods. The liquid crystal media according to the invention preferably contain in addition to components selected from subgroup 1 also components of subgroup 2, the percentage of these components being as follows:
- subgroup 1:: 20 to 90 %, in particular 30 to 90 %
- subgroup 2:: 10 to 50%, in particular 10 to 50 %

In these liquid crystal media the percentages of the compounds according to the invention and the compounds of subgroup 1 and 2 may add up to give 100 %.

The media according to the invention preferably contain 1 to 40 %, in particular 5 to 30 % of the compounds according to the invention. Media containing more than 40 %, in particular 45 to 90 % of the compounds according to the invention are further preferred. The media contain preferably 3, 4 or 5 compounds according to the invention.

The media according to the invention are prepared in a manner which is customary per se. As a rule, the components are dissolved in one another, advantageously at elevated temperature. The liquid crystal media according to the invention can be modified by suitable additives so that they can be used in all the types of liquid crystal display devices. Such additives are known to the expert and are described in detail in the literature (H. Kelker/ R. Hatz, Handbook of Liquid Crystals, Verlag Chemie, Weinheim, 1980). For example, it is possible to add pleochroic dyestuffs to prepare colored guest-host systems or substances for modifying the dielectric anisotropy, the viscosity and/or the orientation of the nematic phases.

The following examples are to be construed as merely illustrative and not limitative. m.p. = melting point, c.p. = clearing point. In the foregoing and in the following all parts and percentages are by weight and the temperatures are set forth in degrees Celsius. "Customary work-up" means that water is added, the mixture is extracted with methylene chloride, the organic phase is separated off, dried and evaporated, and the product is purified by crystallization and/or chromatography.

Further are:
C: crystalline-solid state, S: smectic phase (the index denoting the type of smectic phase), N: nematic phase, Ch: cholesteric phase, I: isotropic phase. The number being embraced by 2 of these symbols denotes the temperature of phase change.

### Example 1

a) 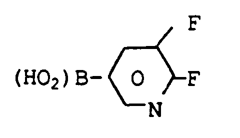 48.5 mM of magnesium in 8 ml of THF is slowly added under dry nitrogen to a mixture of 44.1 mM of 2,3-difluoro-5-bromopyridine (obtainable by reacting 2,3-dichloro-5- nitropyridine with anhydrous KF in DMF at 120°, hydrogenation of the nitro-group and transferring the NH₂-group with NaNO₂ via the diazonium salt and with HBr into the bromo-derivative) in 50 ml of THF. The Grignard reagent is refluxed for a further hour, cooled in ice and 53.4 mM of trimethylborate is added. After stirring overnight the borate ester is hydrolysed with dilute HCl, the product extracted into ether, dried and the solvent removed.
b) 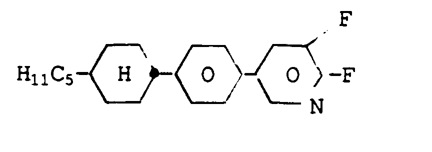 35 mM of 4-(trans-4-pentylcyclohexyl)-bromobenzene, 38.5 mM of 2,3-difluoro-pyridine-5-bromic acid and Pd(PPh₃)₄ (4 x 10⁻⁴ mM) are refluxed overnight under nitrogen, in a mixture of toluene (45 ml), aqueous Na₂CO₃ (20 ml) and 15 ml of ethanol.
   Customary work-up gives 2,3-difluoro-5-[4-(trans-4-pentylcyclohexyl)phenyl]-pyridine.

The following compounds are obtained analogously:
2,3-difluoro-5-[4-(trans-4-ethylcyclohexyl)phenyl]-pyridine
2,3-difluoro-5-[4-(trans-4-propylcyclohexyl)phenyl]-pyridine
2,3-difluoro-5-[4-(trans-4-butylcyclohexyl)phenyl]-pyridine
2,3-difluoro-5-[4-(trans-4-hexylcyclohexyl)phenyl]-pyridine
2,3-difluoro-5-[4-(trans-4-heptylcyclohexyl)phenyl]-pyridine
2,3-dichloro-5-[4-(trans-4-ethylcyclohexyl)phenyl]-pyridine
2,3-dichloro-5-[4-(trans-4-propylcyclohexyl)phenyl]-pyridine
2,3-dichloro-5-[4-(trans-4-butylcyclohexyl)phenyl]-pyridine
2,3-dichloro-5-[4-(trans-4-pentylcyclohexyl)phenyl]-pyridine
2,3-dichloro-5-[4-(trans-4-hexylcyclohexyl)phenyl]-pyridine
2,3-dichloro-5-[4-(trans-4-heptylcyclohexyl)phenyl]-pyridine
2,3-difluoro-5-[4-(trans-4-ethylcyclohexylethyl)phenyl]-pyridine
2,3-difluoro-5-[4-(trans-4-propylcyclohexylethyl)phenyl]-pyridine
2,3-difluoro-5-[4-(trans-4-butylcyclohexylethyl)phenyl]-pyridine
2,3-difluoro-5-[4-(trans-4-pentylcyclohexylethyl)phenyl]-pyridine
2,3-difluoro-5-[4-(trans-4-hexylcyclohexylethyl)phenyl]-pyridine
2,3-difluoro-5-[4-(trans-4-heptylcyclohexylethyl)phenyl]-pyridine
2,3-dichloro-5-[4-(trans-4-ethylcyclohexylethyl)phenyl]-pyridine
2,3-dichloro-5-[4-(trans-4-propylcyclohexylethyl)phenyl]-pyridine
2,3-dichloro-5-[4-(trans-4-butylcyclohexylethyl)phenyl]-pyridine
2,3-dichloro-5-[4-(trans-4-pentylcyclohexylethyl)phenyl]-pyridine
2,3-dichloro-5-[4-(trans-4-hexylcyclohexylethyl)phenyl]-pyridine
2,3-dichloro-5-[4-(trans-4-heptylcyclohexylethyl)phenyl]-pyridine
2,3-difluoro-5-[4-(trans-4-ethylcyclohexyl)-2-fluorophenyl]-pyridine
2,3-difluoro-5-[4-(trans-4-propylcyclohexyl)-2-fluorophenyl]-pyridine
2,3-difluoro-5-[4-(trans-4-butylcyclohexyl)-2-fluorophenyl]-pyridine
2,3-difluoro-5-[4-(trans-4-pentylcyclohexyl)-2-fluorophenyl]-pyridine
2,3-difluoro-5-[4-(trans-4-hexylcyclohexyl)-2-fluorophenyl]-pyridine
2,3-difluoro-5-[4-(trans-4-heptylcyclohexyl)-2-fluorophenyl]-pyridine
2,3-difluoro-5-[4-(trans-4-octylcyclohexyl)-2-fluorophenyl]-pyridine
2,3-dichloro-5-[4-(trans-4-ethylcyclohexyl)-3-fluorophenyl]-pyridine
2,3-dichloro-5-[4-(trans-4-propylcyclohexyl)-3-fluorophenyl]-pyridine
2,3-dichloro-5-[4-(trans-4-butylcyclohexyl)-3-fluorophenyl]-pyridine
2,3-dichloro-5-[4-(trans-4-pentylcyclohexyl)-3-fluorophenyl]-pyridine
2,3-dichloro-5-[4-(trans-4-hexylcyclohexyl)-3-fluorophenyl]-pyridine
2,3-dichloro-5-[4-(trans-4-heptylcyclohexyl)-3-fluorophenyl]-pyridine
2,3-difluoro-5-(4-ethylbiphenyl-4'-yl)-pyridine
2,3-difluoro-5-(4-propylbiphenyl-4'-yl)-pyridine
2,3-difluoro-5-(4-butylbiphenyl-4'-yl)-pyridine
2,3-difluoro-5-(4-pentylbiphenyl-4'-yl)-pyridine
2,3-difluoro-5-(4-hexylbiphenyl-4'-yl)-pyridine
2,3-difluoro-5-(4-heptylbiphenyl-4'-yl)-pyridine
2,3-difluoro-5-(2-fluoro-4-ethylbiphenyl-4'-yl)-pyridine
2,3-difluoro-5-(2-fluoro-4-propylbiphenyl-4'-yl)-pyridine
2,3-difluoro-5-(2-fluoro-4-butylbiphenyl-4'-yl)-pyridine
2,3-difluoro-5-(2-fluoro-4-pentylbiphenyl-4'-yl)-pyridine
2,3-difluoro-5-(2-fluoro-4-hexylbiphenyl-4'-yl)-pyridine
2,3-difluoro-5-(2-fluoro-4-heptylbiphenyl-4'-yl)-pyridine
2,3-dichloro-5-(2'-fluoro-4-ethylbiphenyl-4'-yl)-pyridine
2,3-dichloro-5-(2'-fluoro-4-propylbiphenyl-4'-yl)-pyridine
2,3-dichloro-5-(2'-fluoro-4-butylbiphenyl-4'-yl)-pyridine
2,3-dichloro-5-(2'-fluoro-4-pentylbiphenyl-4'-yl)-pyridine
2,3-dichloro-5-(2'-fluoro-4-hexylbiphenyl-4'-yl)-pyridine
2,3-dichloro-5-(2'-fluoro-4-heptylbiphenyl-4'-yl)-pyridine
2,3-difluoro-5-(4-ethoxybiphenyl-4'-yl)-pyridine
2,3-difluoro-5-(4-propoxybiphenyl-4'-yl)-pyridine
2,3-difluoro-5-(4-butoxybiphenyl-4'-yl)-pyridine
2,3-difluoro-5-(4-pentyloxybiphenyl-4'-yl)-pyridine
2,3-difluoro-5-(4-heptyloxybiphenyl-4'-yl)-pyridine
2,3-difluoro-5-(4-ethylphenyl)-pyridine
2,3-difluoro-5-(4-propylphenyl)-pyridine
2,3-difluoro-5-(4-butylphenyl)-pyridine
2,3-difluoro-5-(4-pentylphenyl)-pyridine
2,3-difluoro-5-(4-hexylphenyl)-pyridine
2,3-difluoro-5-(4-heptylphenyl)-pyridine
2,3-difluoro-5-(4-ethoxyphenyl)-pyridine
2,3-difluoro-5-(4-butoxyphenyl)-pyridine
2,3-difluoro-5-(4-propoxyphenyl)-pyridine
2,3-difluoro-5-(4-pentyloxyphenyl)-pyridine
2,3-dichloro-5-(4-ethylphenyl)-pyridine
2,3-dichloro-5-(4-propylphenyl)-pyridine
2,3-dichloro-5-(4-butylphenyl)-pyridine
2,3-dichloro-5-(4-pentylphenyl)-pyridine
2,3-dichloro-5-(4-hexylphenyl)-pyridine
2,3-dichloro-5-(4-heptylphenyl)-pyridine
2,3-dichloro-5-(4-ethoxyphenyl)-pyridine
2,3-dichloro-5-(4-propoxyphenyl)-pyridine
2,3-dichloro-5-(4-pentyloxyphenyl)-pyridine
2,3-dichloro-5-(4-octyloxyphenyl)-pyridine
2,3-difluoro-5-(2-fluoro-4-ethylphenyl)-pyridine
2,3-difluoro-5-(2-fluoro-4-propylphenyl)-pyridine
2,3-difluoro-5-(2-fluoro-4-butylphenyl)-pyridine
2,3-difluoro-5-(2-fluoro-4-pentylphenyl)-pyridine
2,3-difluoro-5-(2-fluoro-4-hexylphenyl)-pyridine
2,3-difluoro-5-(2-fluoro-4-heptylphenyl)-pyridine

### Example 2

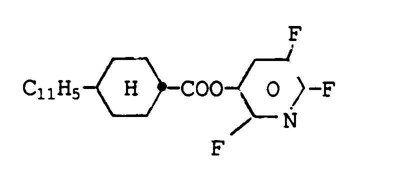

To a mixture of 0.1 M of 2,5,6-trifluoro-3-hydroxypyridine (obtainable by reaction of 2,3,6-trifluoro-pyridine-3-boronic acid with H₂O₂ in ether), 0.1 M of trans-4-pentylcyclohexane carboxylic acid, 0.1 M dimethylaminopyridine (DMAP) and 200 ml of CH₂Cl₂ a solution of 0.11 M of dicyclohexylcarbodiimide (DCC) in CH₂Cl₂ is added at 15-20 °C.

After stirring for 5 hours at room temperature and customary work-up, 2,5,6-trifluoropyridine-3-yl trans-4-pentylcyclohexanecarboxylate is obtained.

The following components are obtained analogously:
2,3,6-trifluoropyridine-5-yl trans-4-ethylcyclohexanecarboxylate
2,3,6-trifluoropyridine-5-yl trans-4-propylcyclohexanecarboxylate
2,3,6-trifluoropyridine-5-yl trans-4-butylcyclohexanecarboxylate
2,3,6-trifluoropyridine-5-yl trans-4-(trans-4-ethylcyclohexyl)-cyclohexanecarboxylate
2,3,6-trifluoropyridine-5-yl trans-4-(trans-4-propylcyclohexyl)-cyclohexanecarboxylate
2,3,6-trifluoropyridine-5-yl trans-4-(trans-4-butylcyclohexyl)-cyclohexanecarboxylate
2,3,6-trifluoropyridine-5-yl trans-4-(trans-4-pentylcyclohexyl)-cyclohexanecarboxylate
2,3-difluoropyridine-5-yl trans-4-(trans-4-ethylcyclohexyl)-cyclohexanecarboxylate
2,3-difluoropyridine-5-yl trans-4-(trans-4-propylcyclohexyl)-cyclohexanecarboxylate
2,3-difluoropyridine-5-yl trans-4-(trans-4-butylcyclohexyl)-cyclohexanecarboxylate
2,3-difluoropyridine-5-yl trans-4-(trans-4-pentylcyclohexyl)-cyclohexanecarboxylate
2,3-difluoropyridine-5-yl trans-4-(trans-4-heptylcyclohexyl)-cyclohexanecarboxylate
2,3-dichloropyridine-5-yl trans-4-(trans-4-ethylcyclohexyl)-cyclohexanecarboxylate
2,3-dichloropyridine-5-yl trans-4-(trans-4-propylcyclohexyl)-cyclohexanecarboxylate
2,3-dichloropyridine-5-yl trans-4-(trans-4-butylcyclohexyl)-cyclohexanecarboxylate
2,3-dichloropyridine-5-yl trans-4-(trans-4-pentylcyclohexyl)-cyclohexanecarboxylate
2,3-dichloropyridine-5-yl trans-4-(trans-4-octylcyclohexyl)-cyclohexanecarboxylate
2,3-difluoro-6-chloropyridine-5-yl trans-4-ethylcyclohexanecarboxylate
2,3-difluoro-6-chloropyridine-5-yl trans-4-propylcyclohexanecarboxylate
2,3-difluoro-6-chloropyridine-5-yl trans-4-butylcyclohexanecarboxylate
2,3-difluoro-6-chloropyridine-5-yl trans-4-pentylcyclohexanecarboxylate
2,3,6-trifluoropyridine-5-yl 4-ethylbenzoate
2,3,6-trifluoropyridine-5-yl 4-propylbenzoate
2,3,6-trifluoropyridine-5-yl 4-butylbenzoate
2,3,6-trifluoropyridine-5-yl 4-pentylbenzoate
2,3,6-trifluoropyridine-5-yl 4-ethoxybenzoate
2,3,6-trifluoropyridine-5-yl 4-propoxybenzoate
2,3,6-trifluoropyridine-5-yl 4-butoxybenzoate
2,3,6-trifluoropyridine-5-yl 4-pentyloxybenzoate
2,3,6-trifluoropyridine-5-yl 4-hexyloxybenzoate
2,3,6-trifluoropyridine-5-yl 4-heptyloxybenzoate
2,3,6-trifluoropyridine-5-yl 4-octyloxybenzoate
2,3-difluoropyridine-5-yl 4-ethylbenzoate
2,3-difluoropyridine-5-yl 4-propylbenzoate
2,3-difluoropyridine-5-yl 4-butylbenzoate
2,3-difluoropyridine-5-yl 4-pentylbenzoate
2,3-difluoropyridine-5-yl 4-ethoxybenzoate
2,3-difluoropyridine-5-yl 4-propoxybenzoate
2,3-difluoropyridine-5-yl 4-butoxybenzoate
2,3-difluoropyridine-5-yl 4-pentyloxybenzoate
2,3-difluoropyridine-5-yl 4-hexyloxybenzoate
2,3-difluoropyridine-5-yl 4-heptyloxybenzoate
2,3-difluoropyridine-5-yl 4-octyloxybenzoate
2,3-difluoropyridine-5-yl trans-4-ethylcyclohexanecarboxylate
2,3-difluoropyridine-5-yl trans-4-propylcyclohexanecarboxylate
2,3-difluoropyridine-5-yl trans-4-butylcyclohexanecarboxylate
2,3-difluoropyridine-5-yl trans-4-pentylcyclohexanecarboxylate
2,3-dichloropyridine-5-yl 4-(4-ethylphenyl)benzoate
2,3-dichloropyridine-5-yl 4-(4-propylphenyl)benzoate
2,3-dichloropyridine-5-yl 4-(4-butylphenyl)benzoate
2,3-dichloropyridine-5-yl 4-(4-pentylphenyl)benzoate
2,3-dichloropyridine-5-yl 4-(4-octylphenyl)benzoate
2,3-difluoro-6-chloropyridine-3-yl 4-(4-ethylphenyl)benzoate
2,3-difluoro-6-chloropyridine-3-yl 4-(4-propylphenyl)benzoate
2,3-difluoro-6-chloropyridine-3-yl 4-(4-butylphenyl)benzoate
2,3-difluoro-6-chloropyridine-3-yl 4-(4-pentylphenyl)benzoate
2,3-difluoro-6-chloropyridine-3-yl 4-(4-heptylphenyl)benzoate
2,3,6-trifluoropyridine-5-yl 4-(trans-4-ethylcyclohexyl)benzoate
2,3,6-trifluoropyridine-5-yl 4-(trans-4-propylcyclohexyl)benzoate
2,3,6-trifluoropyridine-5-yl 4-(trans-4-butylcyclohexyl)benzoate
2,3,6-trifluoropyridine-5-yl 4-(trans-4-pentylcyclohexyl)benzoate
2,3,6-trifluoropyridine-5-yl trans-4-(4-ethylphenyl)cyclohexanecarboxylate
2,3,6-trifluoropyridine-5-yl trans-4-(4-propylphenyl)cyclohexanecarboxylate
2,3,6-trifluoropyridine-5-yl trans-4-(4-butylphenyl)cyclohexanecarboxylate
2,3,6-trifluoropyridine-5-yl trans-4-(4-pentylphenyl)cyclohexanecarboxylate

### Example 3

a) 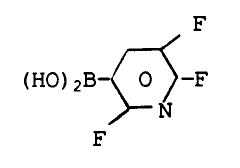 0,6 M of n-butyllithium are added at -10° to 0,6 M of diisopropylamin in 600 ml THF. The mixture is cooled to -70°, 0,6 M of 2,3,6-trifluoropyridin are added and after stirring for 15 minutes 0,6 M of trimethylborate are added at the same temperature. The mixture is allowed to warm up to -30°. Then the borate ester is hydrolysed with dilute HCl, the product extracted with ether, washed, dried and the solvent removed. Thus, 2,3,6-trifluoro-pyridine-5-boronic acid is obtained.
b) 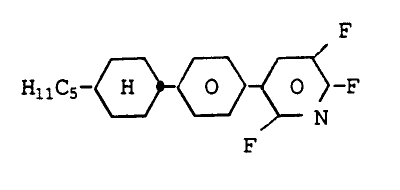 A mixture of 0,05 M of 4-(trans-4-pentylcyclohexyl)bromobenzene, 0,06 M of 2,3,6-trifluoropyridine-5-boronic acid, 1,25 g of Pd(PPh₃)₄, 100 ml of toluene, 40 ml of ethanol and 50 ml of 2 M Na₂CO₃-solution is heated at 60 °C under stirring for 2 hours. Customary work-up and recrystallisation from ethanol gives 2,3,6-trifluoro-5-[4-(trans-4-pentylcyclohexyl)phenyl]-pyridine.

The following compounds are obtained analogously:
2,3,6-trifluoro-5-[4-(trans-4-ethylcyclohexyl)phenyl]-pyridine
2,3,6-trifluoro-5-[4-(trans-4-propylcyclohexyl)phenyl]-pyridine
2,3,6-trifluoro-5-[4-(trans-4-butylcyclohexyl)phenyl]-pyridine
2,3-difluoro-6-chloro-5-[4-(trans-4-ethylcyclohexyl)phenyl]-pyridine
2,3-difluoro-6-chloro-5-[4-(trans-4-propylcyclohexyl)phenyl]-pyridine
2,3-difluoro-6-chloro-5-[4-(trans-4-butylcyclohexyl)phenyl]-pyridine
2,3-difluoro-6-chloro-5-[4-(trans-4-pentylcyclohexyl)phenyl]-pyridine
2,3-difluoro-6-chloro-5-[4-(trans-4-hexylcyclohexyl)phenyl]-pyridine
2,3,6-trifluoro-5-[4-(trans-4-ethylcyclohexylethyl)phenyl]-pyridine
2,3,6-trifluoro-5-[4-(trans-4-propylcyclohexylethyl)phenyl]-pyridine
2,3,6-trifluoro-5-[4-(trans-4-butylcyclohexylethyl)phenyl]-pyridine
2,3,6-trifluoro-5-[4-(trans-4-pentylcyclohexylethyl)phenyl]-pyridine
2,3,6-trifluoro-5-[4-(trans-4-octylcyclohexylethyl)phenyl]-pyridine
2,3-difluoro-6-chloro-5-[4-(trans-4-ethylcyclohexylethyl)phenyl]-pyridine
2,3-difluoro-6-chloro-5-[4-(trans-4-propylcyclohexylethyl)phenyl]-pyridine
2,3-difluoro-6-chloro-5-[4-(trans-4-butylcyclohexylethyl)pheny]l-pyridine
2,3-difluoro-6-chloro-5-[4-(trans-4-pentylcyclohexylethyl)phenyl]-pyridine
2,3,6-trifluoro-5-[4-(trans-4-ethylcyclohexyl)-2-fluorophenyl]-pyridine
2,3,6-trifluoro-5-[4-(trans-4-propylcyclohexyl)-2-fluorophenyl)-pyridine
2,3,6-trifluoro-5-[4-(trans-4-butylcyclohexyl)-2-fluorophenyl]-pyridine
2,3,6-trifluoro-5-[4-(trans-4-pentylcyclohexyl)-2-fluorophenyl]-pyridine
2,3,6-trifluoro-5-[4-(trans-4-heptylcyclohexyl)-2-fluorophenyl]-pyridine
2,3,6-trifluoro-5-(4-ethylbiphenyl-4'-yl)-pyridine
2,3,6-trifluoro-5-(4-propylbiphenyl-4'-yl)-pyridine
2,3,6-trifluoro-5-(4-butylbiphenyl-4'-yl)-pyridine
2,3,6-trifluoro-5-(4-pentylbiphenyl-4'-yl)-pyridine
2,3,6-trifluoro-5-(4-ethoxybiphenyl-4'-yl)-pyridine
2,3,6-trifluoro-5-(4-propoxybiphenyl-4'-yl)-pyridine
2,3,6-trifluoro-5-(4-butoxybiphenyl-4'-yl)-pyridine
2,3,6-trifluoro-5-(4-pentyloxybiphenyl-4'-yl)-pyridine
2,3,6-trifluoro-5-(4-hexyloxybiphenyl-4'-yl)-pyridine
2,3,6-trifluoro-5-(4-heptyloxybiphenyl-4'-yl)-pyridine
2,3,6-trifluoro-5-(4-octyloxybiphenyl-4'-yl)-pyridine
2,3,6-trifluoro-5-(4-ethylphenyl)-pyridine
2,3,6-trifluoro-5-(4-propylphenyl)-pyridine
2,3,6-trifluoro-5-(4-butylphenyl)-pyridine
2,3,6-trifluoro-5-(4-pentylphenyl)-pyridine
2,3-difluoro-6-chloro-5-(4-ethylphenyl)-pyridine
2,3-difluoro-6-chloro-5-(4-propylphenyl)-pyridine
2,3-difluoro-6-chloro-5-(4-butylphenyl)-pyridine
2,3-difluoro-6-chloro-5-(4-pentylphenyl)-pyridine
2,3-difluoro-6-chloro-5-(4-hexylphenyl)-pyridine
2,3,6-trifluoro-5-(4-ethoxyphenyl)-pyridine
2,3,6-trifluoro-5-(4-propoxyphenyl)-pyridine
2,3,6-trifluoro-5-(4-butoxyphenyl)-pyridine
2,3,6-trifluoro-5-(4-pentyloxyphenyl)-pyridine
2,3,6-trifluoro-5-(4-octyloxyphenyl)-pyridine

### Example 4

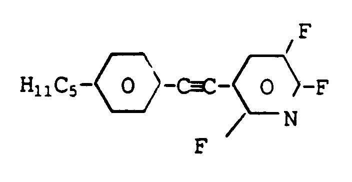

0,2 mM of Pd(Ph₃P)₂Cl₂ and 0,1 mM of CuI are added to a mixture of 0,01 M of 5-bromo-2,3,6-trifluoropyridine (preparation: 2,3,6-trifluoropyridine is reacted with lithiumdiisopropylamide (LDA) as described in example 3a, then Br₂ is added at -60° to -70°. The mixture is allowed to warn up to room temperature and worked up, the product is purified by distillation), 0,01 M of 4-pentylphenylacetylene and 200 ml of triethylamine. The mixture is stirred for 8 hours at room temperature; after customary work-up and purification by crystallization and chromatography 1-(4-pentylphenyl)-2-(2,3,6-trifluoropyridine-5-yl)acetylene is obtained.

The following compounds are obtained analogously:
1-(4-ethylphenyl)-2-(2,3,6-trifluoropyridine-5-yl)acetylene
1-(4-propylphenyl)-2-(2,3,6-trifluoropyridine-5-yl)acetylene
1-(4-butylphenyl)-2-(2,3,6-trifluoropyridine-5-yl)acetylene
1-(4-hexylphenyl)-2-(2,3,6-trifluoropyridine-5-yl)acetylene
1-(4-ethoxyphenyl)-2-(2,3,6-trifluoropyridine-5-yl)acetylene
1-(4-butoxyphenyl)-2-(2,3,6-trifluoropyridine-5-yl)acetylene
1-(4-methoxyphenyl)-2-(2,3,6-trifluoropyridine-5-yl)acetylene
1-(4-propoxyphenyl)-2-(2,3,6-trifluoropyridine-5-yl)acetylene
1-(4-pentyloxyphenyl)-2-(2,3,6-trifluoropyridine-5-yl)acetylene
1-(4-ethylphenyl)-2-(2,3-difluoro-6-chloropyridine-5-yl)-acetylene
1-(4-propylphenyl)-2-(2,3-difluoro-6-chloropyridine-5-yl)-acetylene
1-(4-butylphenyl)-2-(2,3-difluoro-6-chloropyridine-5-yl)acetylene
1-(4-pentylphenyl)-2-(2,3-difluoro-6-chloropyridine-5-yl)acetylene
1-(4-ethylbiphenyl-4'-yl)-2-(2,3,6-trifluoropyridine-5-yl)acetylene
1-(4-propylbiphenyl-4'-yl)-2-(2,3,6-trifluoropyridine-5-yl)acetylene
1-(4-butylbiphenyl-4'-yl)-2-(2,3,6-trifluoropyridine-5-yl)acetylene
1-(4-pentylbiphenyl-4'-yl)-2-(2,3,6-trifluoropyridine-5-yl)acetylene
1-(4-ethoxybiphenyl-4'-yl)-2-(2,3,6-trifluoropyridine-5-yl)-acetylene
1-(4-propoxybiphenyl-4'-yl)-2-(2,3,6-trifluoropyridine-5-yl)-acetylene
1-(4-butoxybiphenyl-4'-yl)-2-(2,3,6-trifluoropyridine-5-yl)-acetylene
1-(4-pentyloxybiphenyl-4'-yl)-2-(2,5,6-trifluoropyridine-5-yl)-acetylene
1-[4-(trans-4-ethylcyclohexyl)phenyl]-2-(2,3,6-trifluoropyridine-5-yl)acetylene
1-[4-(trans-4-propylcyclohexyl)phenyl]-2-(2,3,6-trifluoropyridine-5-yl)acetylene
1-[4-(trans-4-butylcyclohexyl)phenyl]-2-(2,3,6-trifluoropyridine-5-yl)acetylene
1-[4-(trans-4-pentylcyclohexyl)phenyl]-2-(2,3,6-trifluoropyridine-5-yl)acetylene
1-[trans-4-(4-ethylphenyl)cyclohexyl]-2-(2,3,6-trifluoropyridine-5-yl)acetylene
1-[trans-4-(4-propylphenyl)cyclohexyl]-2-(2,3,6-trifluoropyridine-5-yl)acetylene
1-[trans-4-(4-butylphenyl)cyclohexyl]-2-(2,3,6-trifluoropyridine-5-yl)acetylene
1-[trans-4-(4-pentylphenyl)cyclohexyl]-2-(2,3,6-trifluoropyridine-5-yl)acetylene
1-[trans-4-(4-hexylphenyl)cyclohexyl]-2-(2,3,6-trifluoropyridine-5-yl)acetylene
1-[trans-4-(trans-4-ethylcyclohexyl)cyclohexyl]-2-(2,3,6-trifluoropyridine-5-yl)acetylene
1-[trans-4-(trans-4-propylcyclohexyl)cyclohexyl]-2-(2,3,6-trifluoropyridine-5-yl)acetylene
1-[trans-4-(trans-4-butylcyclohexyl)cyclohexyl]-2-(2,3,6-trifluoropyridine-5-yl)acetylene
1-[trans-4-(trans-4-pentylcyclohexyl)cyclohexyl]-2-(2,3,6-trifluoropyridine-5-yl)acetylene
1-[trans-4-(trans-4-heptylcyclohexyl)cyclohexyl]-2-(2,3,6-trifluoropyridine-5-yl)acetylene
1-(trans-4-ethylcyclohexyl)-2-(2,3,6-trifluoropyridine-5-yl)acetylene
1-(trans-4-propylcyclohexyl)-2-(2,3,6-trifluoropyridine-5-yl)acetylene
1-(trans-4-butylcyclohexyl)-2-(2,3,6-trifluoropyridine-5-yl)acetylene
1-(trans-4-pentylcyclohexyl)-2-(2,3,6-trifluoropyridine-5-yl)acetylene

1-[trans-4-(4-ethoxyphenyl)cyclohexyl]-2-(2,3,6-trifluoropyridine-5-yl)acetylene
1-[trans-4-(4-propoxyphenyl)cyclohexyl]-2-(2,3,6-triluoropyridine-5-yl)acetylene
1-[trans-4-(4-butoxyphenyl)cyclohexyl]-2-(2,3,6-trifluoropyridine-5-yl)acetylene
1-[trans-4-(4-pentyloxyphenyl)cyclohexyl]-2-(2,3,6-trifluoropyridine-5-yl)acetylene
1-[trans-4-(4-octyloxyphenyl)cyclohexyl]-2-(2,3,6-trifluoropyridine-5-yl)acetylene
1-[4-(trans-4-ethylcyclohexyl)phenyl]-2-(2,3-difluoro-6-chloropyridine-5-yl)acetylene
1-[4-(trans-4-propylcyclohexyl)phenyl]-2-(2,3-difluoro-6-chloropyridine-5-yl)acetylene
1-[4-(trans-4-butylcyclohexyl)phenyl]-2-(2,3-difluoro-6-chloropyridine-5-yl)acetylene
1-[4-(trans-4-pentylcyclohexyl)phenyl]-2-(2,3-difluoro-6-chloropyridine-5-yl)acetylene
1-(4-ethylphenyl)-2-(2,3-difluoropyridine-5-yl)acetylene
1-(4-propylphenyl)-2-(2,3-difluoropyridine-5-yl)acetylene
1-(4-butylphenyl)-2-(2,3-difluoropyridine-5-yl)acetylene
1-(4-heptylphenyl)-2-(2,3-difluoropyridine-5-yl)acetylene
1-(4-ethoxyphenyl)-2-(2,3-difluoropyridine-5-yl)acetylene
1-(4-propoxyphenyl)-2-(2,3-difluoropyridine-5-yl)acetylene
1-(4-butoxyphenyl)-2-(2,3-difluoropyridine-5-yl)acetylene
1-(4-pentyloxyphenyl)-2-(2,3-difluoropyridine-5-yl)acetylene
1-(4-ethylphenyl)-2-(2,3-dichloropyridin-5-yl)acetylene
1-(4-propylphenyl)-2-(2,3-dichloropyridin-5-yl)acetylene
1-(4-butylphenyl)-2-(2,3-dichloropyridin-5-yl)acetylene
1-(4-pentylphenyl)-2-(2,3-dichloropyridin-5-yl)acetylene
1-(4-ethoxyphenyl)-2-(2,3-dichloropyridine-5-yl)acetylene
1-(4-propoxyphenyl)-2-(2,3-dichloropyridine-5-yl)acetylene
1-(4-butoxyphenyl)-2-(2,3-dichloropyridine-5-yl)acetylene
1-(4-pentyloxyphenyl)-2-(2,3-dichloropyridine-5-yl)acetylene
1-(4-ethylbiphenyl-4'-yl)-2-(2,3-difluoropyridine-5-yl)acetylene
1-(4-propylbiphenyl-4'-yl)-2-(2,3-difluoropyridine-5-yl)acetylene
1-(4-butylbiphenyl-4'-yl)-2-(2,3-difluoropyridine-5-yl)acetylene
1-(4-pentylbiphenyl-4'-yl)-2-(2,3-difluoropyridine-5-yl)acetylene
1-[4-(trans-4-ethylcyclohexyl)phenyl]-2-[2,3-difluoropyridine-5-yl]acetylene
1-[4-(trans-4-propylcyclohexyl)phenyl]-2-[2,3-difluoropyridine-5-yl]acetylene
1-[4-(trans-4-butylcyclohexyl)phenyl]-2-[2,3-difluoropyridine-5-yl]acetylene
1-[4-(trans-4-pentylcyclohexyl)phenyl]-2-[2,3-difluoropyridine-5-yl]acetylene
1-[4-(trans-4-octylcyclohexyl)phenyl]-2-[2,3-difluoropyridine-5-yl]acetylene

1-[trans-4-(4-ethylphenyl)cyclohexyl]-2-(2,3-dichloropyridine-5-yl)acetylene
1-[trans-4-(4-propylphenyl)cyclohexyl]-2-(2,3-dichloropyridine-5-yl)acetylene
1-[trans-4-(4-butylphenyl)cyclohexyl]-2-(2,3-dichloropyridine-5-yl)acetylene
1-[trans-4-(4-pentylphenyl)cyclohexyl]-2-(2,3-dichloropyridine-5-yl)acetylene
1-[trans-4-(4-ethoxyphenyl)cyclohexyl]-2-(2,3-difluoropyridine-5-yl)acetylene
1-[trans-4-(4-propoxyphenyl)cyclohexyl]-2-(2,3-difluoropyridine-5-yl)acetylene
1-[trans-4-(4-butoxyphenyl)cyclohexyl]-2-(2,3-difluoropyridine-5-yl)acetylene
1-[trans-4-(4-pentyloxyphenyl)cyclohexyl1-2-(2,3-difluoropyridine-5-yl)acetylene
1-(trans-4-ethylcyclohexyl)-2-(2,3-difluoropyridine-5-yl)acetylene
1-(trans-4-propylcyclohexyl)-2-(2,3-difluoropyridine-5-yl)acetylene
1-(trans-4-butylcyclohexyl)-2-(2,3-difluoropyridine-5-yl)acetylene
1-(trans-4-pentylcyclohexyl)-2-(2,3-difluoropyridine-5-yl)acetylene
1-[trans-4-(trans-4-ethylcyclohexyl)cyclohexyl]-2-(2,3-difluoropyridine-5-yl)acetylene
1-[trans-4-(trans-4-propylcyclohexyl)cyclohexyl)-2-(2,3-difluoropyridine-5-yl)acetylene
1-[trans-4-(trans-4-butylcyclohexyl)cyclohexyl]-2-(2,3-difluoropyridine-5-yl)acetylene
1-[trans-4-(trans-4-pentylcyclohexyl)cyclohexyl]-2-(2,3-difluoropyridine-5-yl)acetylene

### Example 5

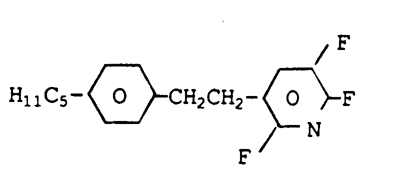

Hydrogenation of 1-(4-pentylphenyl)-2-(2,3,6-trifluoro-pyridine-5o-yl)acetylene yields after customary work-up 1-(4-pentylphenyl)-2-(2,3,6-trifluoropyridine-5-yl)ethane.

The following compounds are obtained analogously:
1-(4-ethylphenyl)-2-(2,3,6-trifluoropyridine-5-yl)ethane
1-(4-propylphenyl)-2-(2,3,6-trifluoropyridine-5-yl)ethane
1-(4-butylphenyl)-2-(2,3,6-trifluoropyridine-5-yl)ethane
1-(4-hexylphenyl)-2-(2,3,6-trifluoropyridine-5-yl)ethane
1-(4-ethoxyphenyl)-2-(2,3,6-trifluoropyridine-5-yl)ethane
1-(4-butoxyphenyl)-2-(2,3,6-trifluoropyridine-5-yl)ethane
1-(4-methoxyphenyl)-2-(2,3,6-trifluoropyridine-5-yl)ethane
1-(4-propoxyphenyl)-2-(2,3,6-trifluoropyridine-5-yl)ethane
1-(4-pentyloxyphenyl)-2-(2,3,6-trifluoropyridine-5-yl)ethane
1-(4-ethylphenyl)-2-(2,3-difluoro-6-chlorpyridine-5-yl)ethane
1-(4-propylphenyl)-2-(2,3-difluoro-6-chlorpyridine-5-yl)ethane
1-(4-butylphenyl)-2-(2,3-difluoro-6-chlorpyridine-5-yl)ethane
1-(4-pentylphenyl)-2-(2,3-difluoro-6-chlorpyridine-5-yl)ethane
1-(4-ethylbiphenyl-4'-yl)-2-(2,3,6-trifluoropyridine-5-yl)-ethane
1-(4-propylbiphenyl-4'-yl)-2-(2,3,6-trifluoropyridine-5-yl)-ethane
1-(4-butylbiphenyl-4'-yl)-2-(2,3,6-trifluoropyridine-5-yl)-ethane
1-(4-pentylbiphenyl-4'-yl)-2-(2,3,6-trifluoropyridine-5-yl)-ethane
1-(4-ethoxybiphenyl-4'-yl)-2-(2,3,6-trifluoropyridine-5-yl)-ethane
1-(4-propoxybiphenyl-4'-yl)-2-(2,3,6-trifluoropyridine-5-yl)-ethane
1-(4-butoxybiphenyl-4'-yl)-2-(2,3,6-trifluoropyridine-5-yl)-ethane
1-(4-pentyloxybiphenyl-4'-yl)-2-(2,3,6-trifluoropyridine-5-yl)-ethane
1-[4-(trans-4-ethylcyclohexyl)phenyl]-2-(2,3,6-trifluoropyridine-5-yl)ethane
1-[4-(trans-4-propylcyclohexyl)phenyl]-2-(2,3,6-trifluoropyridine-5-yl)ethane
1-[4-(trans-4-butylcyclohexyl)phenyl]-2-(2,3,6-trifluoropyridine-5-yl)ethane
1-[4-(trans-4-pentylcyclohexyl)phenyl]-2-(2,3,6-trifluoropyridine-5-yl)ethane
1-[trans-4-(4-ethylphenyl)cyclohexyl]-2-(2,3,6-trifluoropyridine-5-yl)ethane
1-[trans-4-(4-propylphenyl)cyclohexyl]-2-(2,3,6-trifluoropyridine-5-yl)ethane
1-[trans-4-(4-butylphenyl)cyclohexyl]-2-(2,3,6-trifluoropyridine-5-yl)ethane
1-[trans-4-(4-pentylphenyl)cyclohexyl]-2-(2,3,6-trifluoropyridine-5-yl)ethane
1-[trans-4-(4-hexylphenyl)cyclohexyl]-2-(2,3,6-trifluoropyridine-5-yl)ethane
1-[trans-4-(trans-4-ethylcyclohexyl)cyclohexyl]-2-(2,3,6-trifluoropyridine-5-yl)ethane
1-[trans-4-(trans-4-propylcyclohexyl)cyclohexyl]-2-(2,3,6-trifluoropyridine-5-yl)ethane
1-[trans-4-(trans-4-butylcyclohexyl)cyclohexyl]-2-(2,3,6-trifluoropyridine-5-yl)ethane
1-[trans-4-(trans-4-pentylcyclohexyl)cyclohexyl)-2-(2,3,6-trifluoropyridine-5-yl)ethane
1-[trans-4-(trans-4-heptylcyclohexyl)cyclohexyl]-2-(2,3,6-trifluoropyridine-5-yl)ethane
1-(trans-4-ethylcyclohexyl)-2-(2,3,6-trifluoropyridine-5-yl)ethane
1-(trans-4-propylcyclohexyl)-2-(2,3,6-trifluoropyridine-5-yl)ethane
1-(trans-4-butylcyclohexyl)-2-(2,3,6-trifluoropyridine-5-yl)ethane
1-(trans-4-pentylcyclohexyl)-2-(2,3,6-trifluoropyridine-5-yl)ethane
1-[trans-4-(4-ethoxyphenyl)cyclohexyl]-2-(2,3,6-trifluoropyridine-5-yl)ethane
1-[trans-4-(4-propoxyphenyl)cyclohexyl]-2-(2,3,6-trifluoropyridine-5-yl)ethane
1-[trans-4-(4-butoxyphenyl)cyclohexyl]-2-(2,3,6-trifluoropyridine-5-yl)ethane
1-[trans-4-(4-pentyloxyphenyl)cyclohexyl]-2-(2,3,6-trifluoropyridine-5-yl)ethane
1-[trans-4-(4-octyloxyphenyl)cyclohexyl]-2-(2,3,6-trifluoropyridine-5-yl)ethane
1-[4-(trans-4-ethylcyclohexyl)phenyl]-2-(2,3-difluoro-6-2,3-dichloropyridine-5-yl)ethane
1-[4-(trans-4-propylcyclohexyl)phenyl]-2-(2,3-difluoro-6-2,3-dichloropyridine-5-yl)ethane
1-[4-(trans-4-butylcyclohexyl)phenyl]-2-(2,3-difluoro-6-2,3-dichloropyridine-5-yl)ethane
1-[4-(trans-4-pentylcyclohexyl)phenyl]-2-(2,3-difluoro-6-2,3-dichloropyridine-5-yl)ethane
1-(4-ethylphenyl)-2-(2,3-difluoropyridine-5-yl)ethane
1-(4-propylphenyl)-2-(2,3-difluoropyridine-5-yl)ethane
1-(4-butylphenyl)-2-(2,3-difluoropyridine-5-yl)ethane
1-(4-heptylphenyl)-2-(2,3-difluoropyridine-5-yl)ethane
1-(4-ethoxyphenyl)-2-(2,3-difluoropyridine-5-yl)ethane
1-(4-propoxyphenyl)-2-(2,3-difluoropyridine-5-yl)ethane
1-(4-butoxyphenyl)-2-(2,3-difluoropyridine-5-yl)ethane
1-(4-pentyloxphenyl)-2-(2,3-difluoropyridine-5-yl)ethane
1-(4-ethylphenyl)-2-(2,3-dichloropyridin-5-yl)ethane
1-(4-propylphenyl)-2-(2,3-dichloropyridin-5-yl)ethane
1-(4-butylphenyl)-2-(2,3-dichloropyridin-5-yl)ethane
1-(4-pentylphenyl)-2-(2,3-dichloropyridin-5-yl)ethane
1-(4-ethoxyphenyl)-2-(2,3-dichloropyridine-5-yl)ethane
1-(4-propoxyphenyl)-2-(2,3-dichloropyridine-5-yl)ethane
1-(4-butoxyphenyl)-2-(2,3-dichloropyridine-5-yl)ethane
1-(4-pentyloxyphenyl)-2-(2,3-dichloropyridine-5-yl)ethane
1-(4-ethylbiphenyl-4'-yl)-2-(2,3-difluoropyridine-5-yl)ethane
1-(4-propylbiphenyl-4'-yl)-2-(2,3-difluoropyridine-5-yl)ethane
1-(4-butylbiphenyl-4'-yl)-2-(2,3-difluoropyridine-5-yl)ethane
1-(4-pentylbiphenyl-4'-yl)-2-(2,3-difluoropyridine-5-yl)ethane
1-[4-(trans-4-ethylcyclohexyl)phenyl]-2-[2,3-difluoropyridine-5-y]lethane
1-[4-(trans-4-propylcyclohexyl)phenyl]-2-[2,3-difluoropyridine-5-yl]ethane
1-[4-(trans-4-butylcyclohexyl)phenyl]-2-[2,3-difluoropyridine-5-yl]ethane
1-[4-(trans-4-pentylcyclohexyl)phenyl]-2-[2,3-difluoropyridine-5-yl]ethane
1-[4-(trans-4-octylcyclohexyl)phenyl]-2-[2,3-difluoropyridine-5-yl]ethane
1-[trans-4-(4-ethylphenyl)cyclohexyl]-2-(2,3-dichloro-pyridine-5-yl)ethane
1-[trans-4-(4-propylphenyl)cyclohexyl]-2-(2,3-dichloro-pyridine-5-yl)ethane
1-[trans-4-(4-butylphenyl)cyclohexyl]-2-(2,3-dichloro-pyridine-5-yl)ethane
1-[trans-4-(4-pentylphenyl)cyclohexyl)-2-(2,3-dichloro-pyridine-5-yl)ethane
1-[trans-4-(4-ethoxyphenyl)cyclohexyl]-2-(2,3-difluoropyridine-5-yl)ethane
1-[trans-4-(4-propoxyphenyl)cyclohexyl]-2-(2,3-difluoropyridine-5-yl)ethane
1-[trans-4-(4-butoxyphenyl)cyclohexyl]-2-(2,3-difluoropyridine-5-yl)ethane
1-[trans-4-(4-pentyloxyphenyl)cyclohexyl]-2-(2,3-difluoropyridine-5-yl)ethane
1-(trans-4-ethylcyclohexyl)-2-(2,3-difluoropyridine-5-yl)ethane
1-(trans-4-propylcyclohexyl)-2-(2,3-difluoropyridine-5-yl)ethane
1-(trans-4-butylcyclohexyl)-2-(2,3-difluoropyridine-5-yl)ethane
1-(trans-4-pentylcyclohexyl)-2-(2,3-difluoropyridine-5-yl)ethane
1-[trans-4-(trans-4-ethylcyclohexyl)cyclohexyl]-2-(2,3-difluoropyridine-5-yl)ethane
1-[trans-4-(trans-4-propylcyclohexyl)cyclohexyl]-2-(2,3-difluoropyridine-5-yl)ethane 1-[trans-4-(trans-4-butylcyclohexyl)cyclohexyl]-2-(2,3-difluoropyridine-5-yl)ethane
1-[trans-4-(trans-4-pentylcyclohexyl)cyclohexyl]-2-(2,3-difluoropyridine-5-yl)ethane
1-(trans-4-ethylcyclohexyl)-2-[4-(2,3-dichloropyridin-5-yl)phenyl]-ethane
1-(trans-4-propylcyclohexyl)-2-[4-(2,3-dichloropyridin-5-yl)phenyl]-ethane
1-(trans-4-butylcyclohexyl)-2-[4-(2,3-dichloropyridin-5-yl)phenyl]-ethane
1-(trans-4-pentylcyclohexyl)-2-[4-(2,3-dichloropyridin-5-yl)phenyl]-ethane
1-(trans-4-hexylcyclohexyl)-2-[4-(2,3-dichloropyridin-5-yl)phenyl]-ethane
1-(trans-4-heptylcyclohexyl)-2-[4-(2,3-dichloropyridin-5-yl)phenyl]-ethane

### Example 6

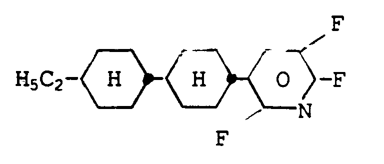

A suspension of 0,02 M of trans-4-(trans-4-ethylcyclohexyl)cyclohexylbromide, 0,04 M of lithium and 0,01 M of ZnBr₂ is stirred for 2 hours at about 10° with ultrasonic. 0,02 M of 5-bromo-2,3,6-trifluoropyridine and 0,1 g of Pd(Ph₃P)₄ are added and the mixture is stirred for 4 hours.

After customary work-up 2,3,6-trifluoro-5-trans-4-(trans-4-ethylcyclohexyl)cyclohexylpyridine is obtained.

The following compounds are obtained analogously:
2,3,6-trifluoro-5-trans-4-(trans-4-methylcyclohexyl)-cyclohexylpyridine
2,3,6-trifluoro-5-trans-4-(trans-4-propylcyclohexyl)-cyclohexylpyridine
2,3,6-trifluoro-5-trans-4-(trans-4-butylcyclohexyl)-cyclohexylpyridine
2,3,6-trifluoro-5-trans-4-(trans-4-pentylcyclohexyl)-cyclohexylpyridine
6-chloro-2,3-difluoro-3-[trans-4-(trans-4-ethylcyclohexyl)cyclohexyl]pyridine
6-chloro-2,3-difluoro-3-[trans-4-(trans-4-propylcyclohexyl)cyclohexyl]pyridine
6-chloro-2,3-difluoro-3-[trans-4-(trans-4-butylcyclohexyl)cyclohexyl]pyridine
6-chloro-2,3-difluoro-3-[trans-4-(trans-4-pentylcyclohexyl)cyclohexyl]pyridine
2,3,6-trifluoro-5-(trans-4-ethylcyclohexyl)pyridine
2,3,6-trifluoro-5-(trans-4-propylcyclohexyl)pyridine
2,3,6-trifluoro-5-(trans-4-butylcyclohexyl)pyridine
2,3,6-trifluoro-5-(trans-4-pentylcyclohexyl)pyridine
2,3,6-trifluoro-5-[trans-4-(4-ethylphenyl)cyclohexyl]pyridine
2,3,6-trifluoro-5-[trans-4-(4-propylphenyl)cyclohexyl]pyridine
2,3,6-trifluoro-5-[trans-4-(4-butylphenyl)cyclohexyl]pyridine
2,3,6-trifluoro-5-[trans-4-(4-pentylphenyl)cyclohexyl]pyridine
2,3,6-trifluoro-5-[trans-4-(4-ethoxyphenyl)cyclohexyl]pyridine
2,3,6-trifluoro-5-[trans-4-(4-propoxyphenyl)cyclohexyl]pyridine
2,3,6-trifluoro-5-[trans-4-(4-butoxyphenyl)cyclohexyl]pyridine
2,3,6-trifluoro-5-[trans-4-(4-pentyloxyphenyl)cyclohexyl]pyridine
2,3,6-trifluoro-5-[trans-4-(4-octyloxyphenyl)cyclohexyl]pyridine
2,3-difluoro-6-chloro-5-[trans-4-(4-ethylphenyl)cyclohexyl]pyridine
2,3-difluoro-6-chloro-5-[trans-4-(4-propylphenyl)cyclohexyl]pyridine
2,3-difluoro-6-chloro-5-[trans-4-(4-butylphenyl)cyclohexyl]pyridine
2,3-difluoro-6-chloro-5-[trans-4-(4-pentylphenyl)cyclohexyl]pyridine
2,3,6-trifluoro-5-[trans-4-(2-fluoro-4-ethylphenyl)cyclohexyl]pyridine
2,3,6-trifluoro-5-[trans-4-(2-fluoro-4-propylphenyl)cyclohexyl]pyridine
2,3,6-trifluoro-5-[trans-4-(2-fluoro-4-butylphenyl)cyclohexyl]pyridine
2,3,6-trifluoro-5-[trans-4-(2-fluoro-4-pentylphenyl)cyclohexyl]pyridine
2,3-difluoro-5-[trans-4-(trans-4-propylcyclohexyl)cyclohexyl]-pyridine
2,3-difluoro-5-[trans-4-(trans-4-butylcyclohexyl)cyclohexyl]-pyridine
2,3-difluoro-5-[trans-4-(trans-4-pentylcyclohexyl)cyclohexyl]-pyridine
2,3-difluoro-5-[trans-4-(trans-4-octylcyclohexyl)cyclohexyl]-pyridine
2,3-dichloro-5-[trans-4-(trans-4-ethylcyclohexyl)cyclohexyl]-pyridine
2,3-dichloro-5-[trans-4-(trans-4-propylcyclohexyl)cyclohexyl]-pyridine
2,3-dichloro-5-[trans-4-(trans-4-butylcyclohexyl)cyclohexyl]-pyridine
2,3-dichloro-5-[trans-4-(trans-4-pentylcyclohexyl)cyclohexyl]-pyridine
2,3-difluoro-5-(trans-4-ethylcyclohexyl)-pyridine
2,3-difluoro-5-(trans-4-propylcyclohexyl)-pyridine
2,3-difluoro-5-(trans-4-butylcyclohexyl)-pyridine
2,3-difluoro-5-(trans-4-pentylcyclohexyl)-pyridine
2,3-difluoro-5-(trans-4-hexylcyclohexyl)-pyridine
2,3-difluoro-5-[trans-4-(4-ethylphenyl)cyclohexyl]pyridine
2,3-difluoro-5-[trans-4-(4-propylphenyl)cyclohexyl]pyridine
2,3-difluoro-5-[trans-4-(4-butylphenyl)cyclohexyl]pyridine
2,3-difluoro-5-[trans-4-(4-pentylphenyl)cyclohexyl]pyridine
2,3-difluoro-5-[trans-4-(4-ethoxyphenyl)cyclohexyl]pyridine
2,3-difluoro-5-[trans-4-(4-propoxyphenyl)cyclohexyl]pyridine
2,3-difluoro-5-[trans-4-(4-butoxyphenyl)cyclohexyl]pyridine
2,3-difluoro-5-[trans-4-(4-pentyloxyphenyl)cyclohexyl]pyridine
2,3-dichloro-5-[trans-4-(4-ethylphenyl)cyclohexyl]pyridine
2,3-dichloro-5-[trans-4-(4-propylphenyl)cyclohexyl]pyridine
2,3-dichloro-5-[trans-4-(4-butylphenyl)cyclohexyl]pyridine
2,3-dichloro-5-[trans-4-(4-pentylphenyl)cyclohexyl]pyridine

### Example 7

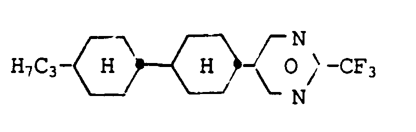

0.1 m trifluoroacetamidine hydrochloride, 0.2 m 1-formyl-1-[trans-(4-propylcyclohexyl)-cyclohexyl]-2-2,2-diethoxyethane (obtainable by Rosenmund Reduction of trans-4-(trans-4-propylcyclohexyl)-cyclohexyl-acetyl chloride, followed by the reaction of the aldehyde with piperidine to the enamine and BF₃-catalyzed additon of triethyl orthoformate to the double bond and hydrogenation) and 50 ml dimethylformamide are refluxed at 140 °C for 22 h. After destillation of the dimethylformamde in vacuo the customary work-up gives 2-trifluoromethyl-5-[trans-4-(trans-4-propyl-cyclohexyl)-cyclohexyl]pyrimidine.

The following compounds are obtained analogously:
2-trifluoromethyl-5-[trans-4-(trans-4-ethyl-cyclohexyl)cyclohexyl]-pyrimidine
2-trifluoromethyl-5-[trans-4-(trans-4-butyl-cyclohexyl)cyclohexyl]-pyrimidine
2-trifluoromethyl-5-[trans-4-(trans-4-pentyl-cyclohexyl)cyclohexyl]-pyrimidine
2-trifluoromethyl-5-[trans-4-(trans-4-hexyl-cyclohexyl)cyclohexyl]-pyrimidine
2-trifluoromethyl-5-[trans-4-(trans-4-heptyl-cyclohexyl)cyclohexyl]-pyrimidine
2-trifluoromethyl-5-[trans-4-(trans-4-ethoxy-cyclohexyl)cyclohexyl]-pyrimidine
2-trifluoromethyl-5-[trans-4-(trans-4-propoxy-cyclohexyl)cyclohexyl]-pyrimidine
2-trifluoromethyl-5-[trans-4-(trans-4-butoxy-cyclohexyl)cyclohexyl]-pyrimidine
2-trifluoromethyl-5-[trans-4-(trans-4-pentyloxy-cyclohexyl)cyclohexyl]-pyrimidine
2-trifluoromethyl-5-[trans-4-(trans-4-heptyloxy-cyclohexyl)cyclohexyl]-pyrimidine

### Example 8

a) 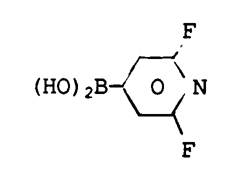 0.6 M of n-butyllithium are added at -10 °C to 0.6 M of diisopropylamin in 600 ml THF. The mixture is cooled to -70 °C, 0.6 M of 2,6-difluoro-4-iodopyridin (prepared according to page 15) are added and after stirring for 15 minutes 0.6 M of trimethylborate are added at the same temperature. The mixture is allowed to warm up to -30 °C. Then the borate ester is hydrolyzed with dilute HCl, the product extracted with ether, washed, dried and the solvent removed. Thus the 2,6-difluoro-pyridine-4-boronic acid is obtained.
b) 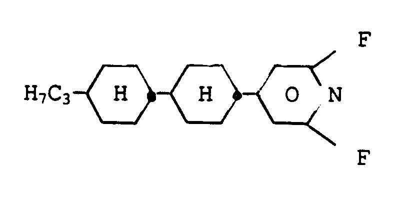 70 mM of trans-4-(trans-4-propylcyclohexyl)-cyclohexylbromide 77 mM of 2,6-difluoro-pyridine-4-boronic acid and 8 · 10⁻⁴ M Pd(PPh₃)₄ are refluxed overnight under nitrogen, in a mixture of 90 ml toluene, 40 ml aqueous Na₂CO₃ and 30 ml of ethanol. Customary work-up gives 2,6-difluoro-4-(trans-4-propylbicyclohexyl)-pyridine.

The following compounds of the formula 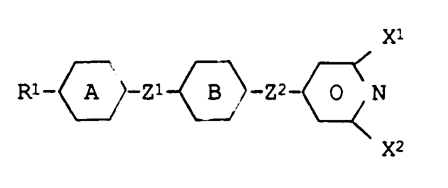 are obtained analogously:

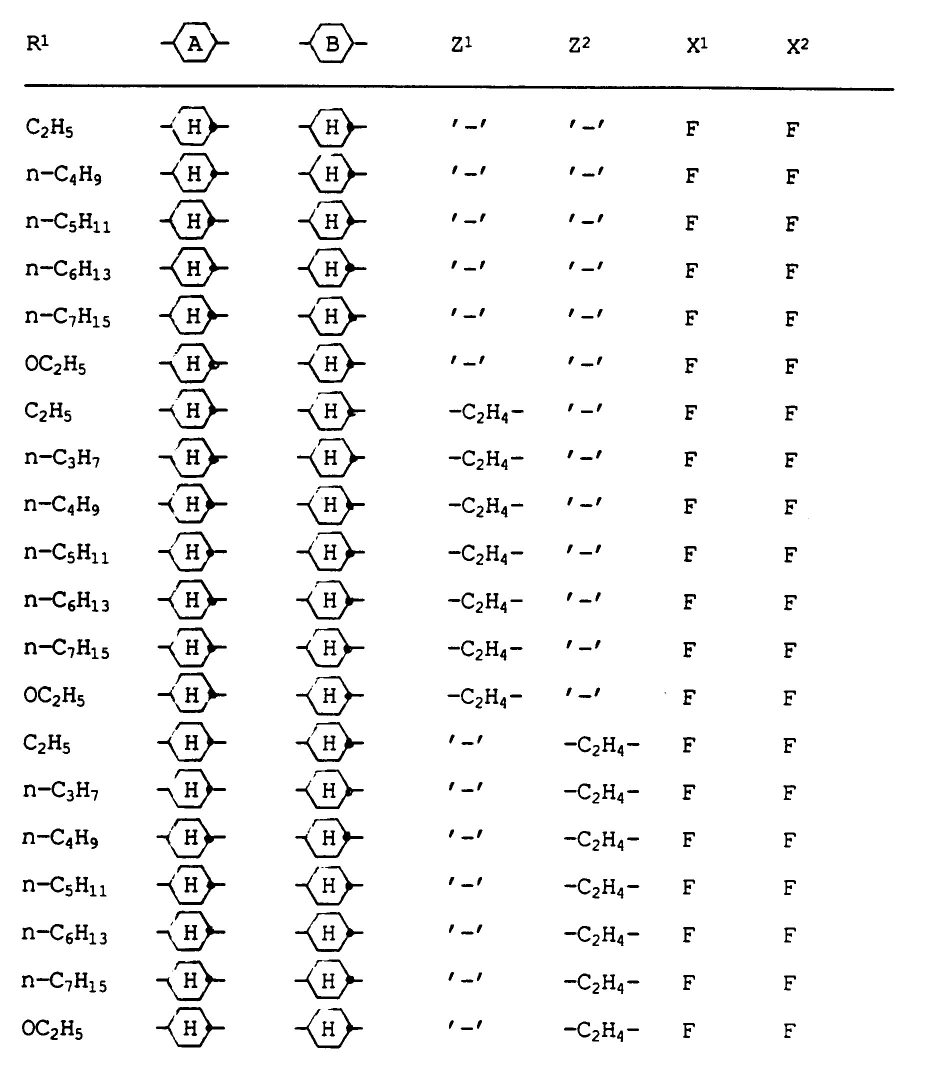

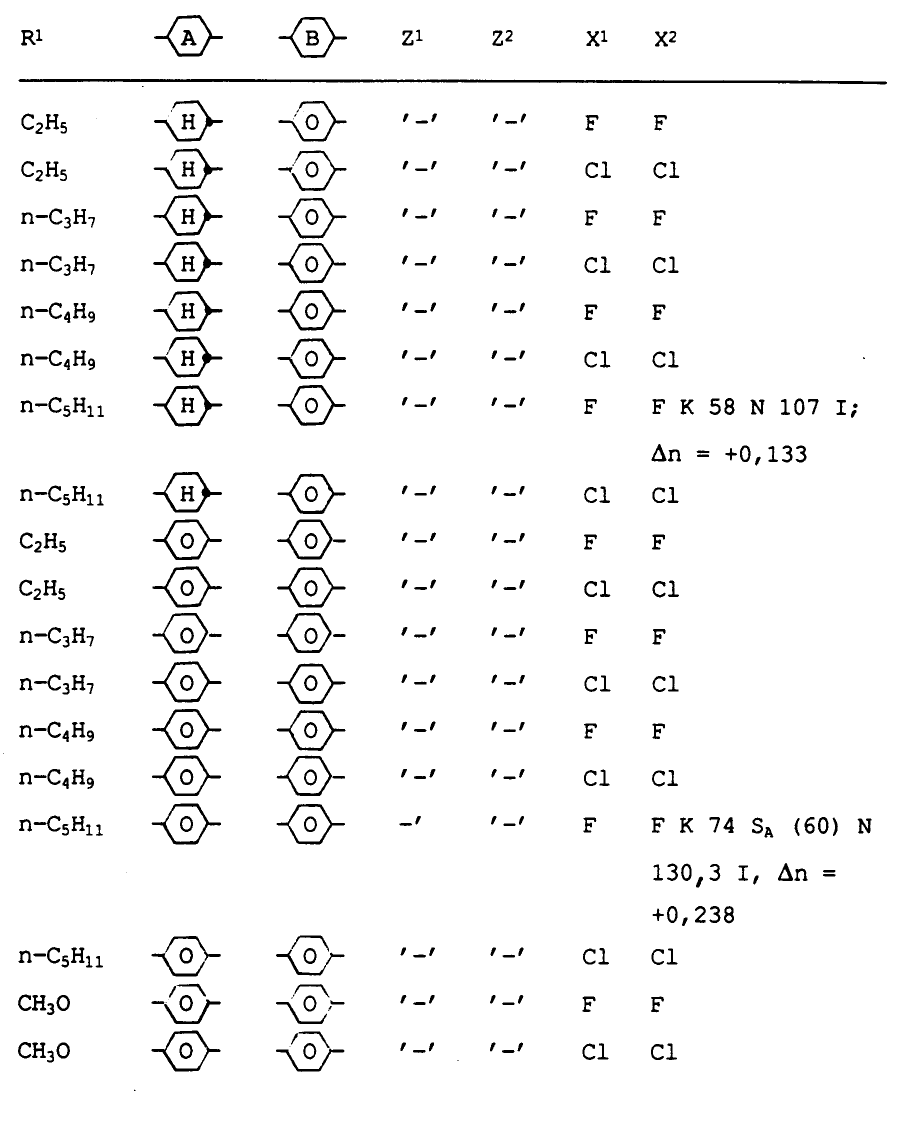

### Comparative Example

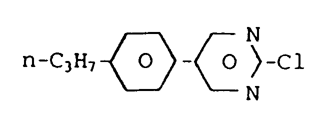

A mixture of 0,05 M of 4-propylbromobenzene, 0,06 M of 2-Chloro-pyrimidine 5-boronic acid (prepared according to page 15), 1.25 g of Pd(PPh₃)₄, 100 ml of toluene, 40 ml of ethanol and 50 ml of 2 M Na₂CO₃ solution is heated at 60 °C under stirring for 2 h: Customary work-up and recrystallization from ethanol gives 2-chloro-5-(4-propylphenyl)-pyrimidine. K 133 I

The following compounds of the formula 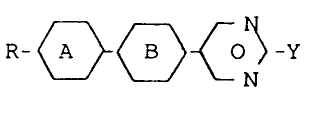 are prepared analogously:

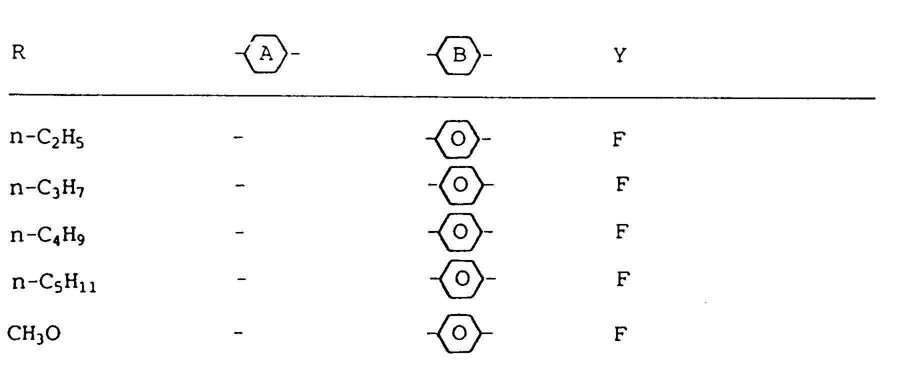

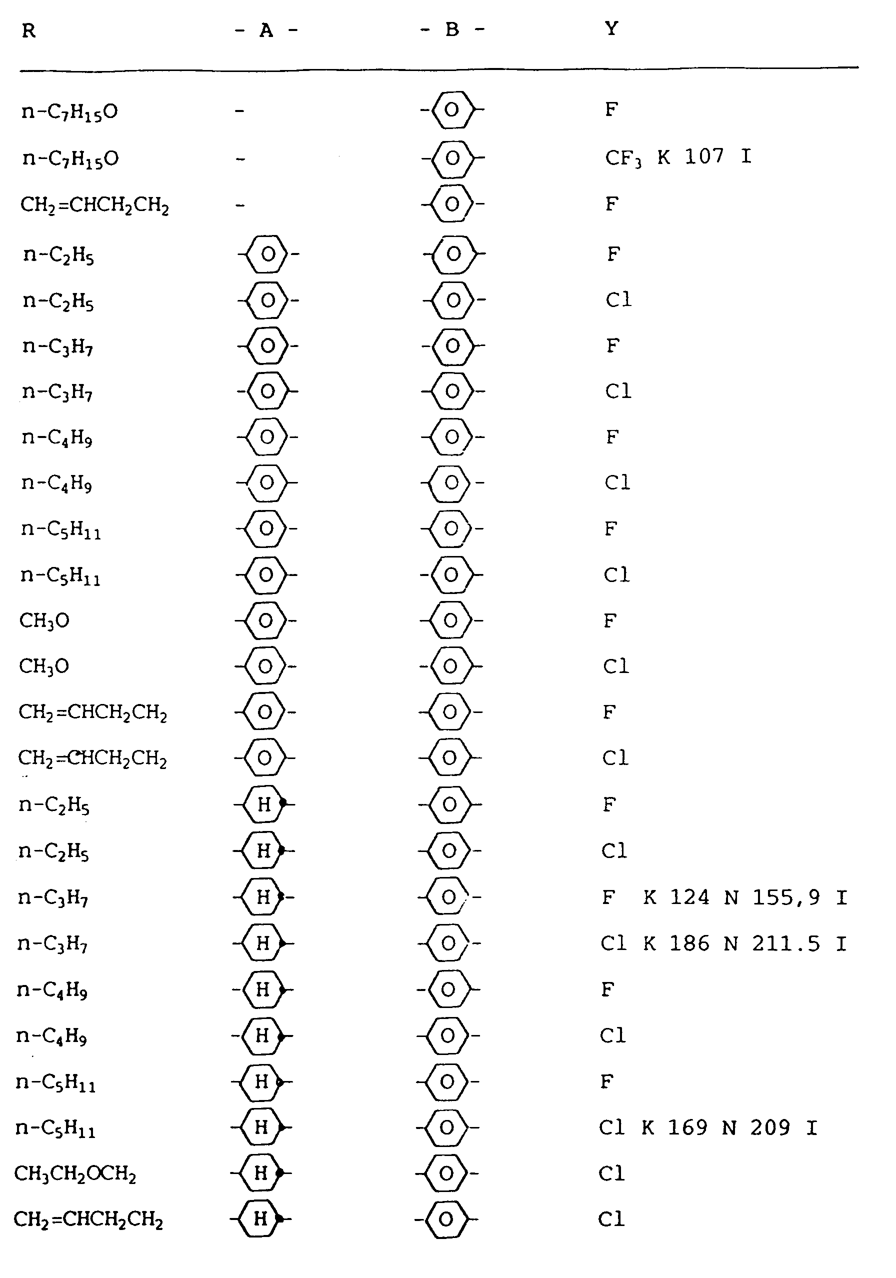

## Claims

1. Heterocyclic derivatives of the formula I 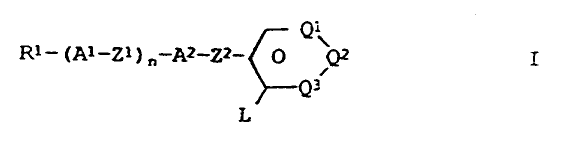 wherein
R¹ denotes alkyl with up to 12 carbon atoms wherein one or two non-adjacent CH₂ groups may also be replaced by -O-, -O-CO-, -CO-O-and/or -CH=CH-,
L is H or F,
A¹ and A² in each case independently of one another are trans-1,4-cyclohexylene, wherein one or two non-adjacent CH₂ groups may also be replaced by -O- and/or -S-, or 1,4-phenylene which is unsubstituted or substituted by one or more halogen atoms and/or nitrile and/or CH₃ groups, and wherein one or more CH groups may also be replaced by N,
Z¹ and Z² in each case independently of one another are -CH₂CH₂-, -CO-O-, -O-CO-, -CH₂O-, -OCH₂-, -C≡C-or a single bond,
n is 0, 1 or 2,
one or two non-adjacent Q's denote N and the remaining bivalent groups Q¹, Q² and Q³ are C-halogen and
in the case that Q¹ and Q³ are N Q² can also be C-O(CF₂)ₛ-Y or C-(CF₂)ₛ-Y, wherein
Y is H, F or Cl, and
s is 1 or 2,
with the exception of compounds of formula I where L is H, N is 0, Q¹ and Q² are N and Q³ is CCl.

2. Heterocyclic derivatives of claim 1, characterized by the formula

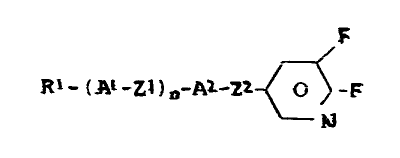

3. Heterocyclic derivatives of claim 1, characterized by the formula

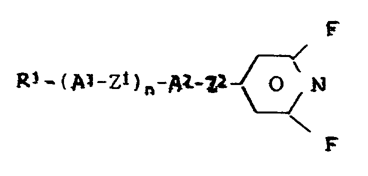

4. Heterocyclic derivatives of claims 1, characterized by the formula

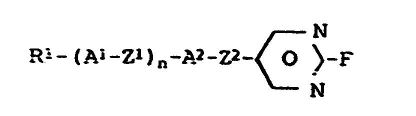

5. Heterocyclic derivatives of claim 1, characterized by the formula

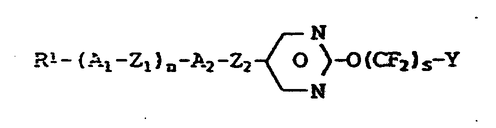

6. Heterocyclic derivatives of claim 1, characterized by the formula

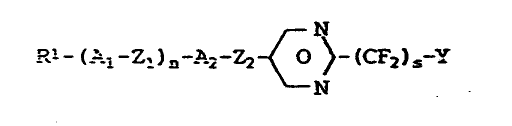

7. Heterocyclic derivatives of claims 1 to 6, wherein R¹ denotes an unbranched alkyl radical of 2 to 7 carbon atoms.

8. Heterocyclic derivatives of claims 1 to 7, wherein n is 0 or 1.

9. Heterocyclic derivatives of claims 1 to 8, wherein Z¹ and Z² are each a single bond.

10. Liquid crystalline medium being a mixture of at least two compounds, characterized in that at least one compound is a heterocyclic derivative according to claim 1.

11. Liquid crystal display device, characterized in that it contains a liquid crystalline medium according to claim 10.

12. Electrooptical display device, characterized in that it contains a liquid crystalline medium according to claim 10.

13. Use of Heterocyclic derivatives characterized by the formula 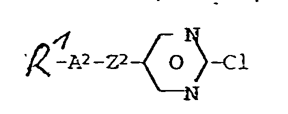 wherein R¹, A² and Z² are defined as in claim 1, in liquid crystalline media.

## Patentansprüche

1. Heterocyclische Derivate der Formel I, 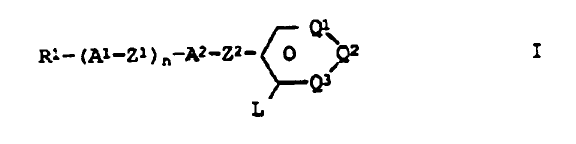 worin
R¹ Alkyl mit bis zu 12 Kohlenstoffatomen, worin eine oder zwei nichtbenachbarte CH₂-Gruppen auch durch -O-, -O-CO-, -CO-O- und/oder -CH=CH- ersetzt sein können,
L H oder F,
A¹ und A² jeweils unabhängig voneinander trans-1,4-Cyclohexylen, worin eine oder zwei nichtbenachbarte CH₂-Gruppen auch durch -O- und/oder -S-ersetzt sein können, oder 1,4-Phenylen, das gegebenenfalls durch ein oder mehrere Halogenatome und/oder Nitril- und/oder CH₃-Gruppen substituiert sein kann und worin eine oder mehrere CH-Gruppen auch durch N ersetzt sein können,
Z¹ und Z² jeweils unabhängig voneinander -CH₂CH₂-, -CO-O-, -O-CO-, -CH₂O-, -OCH₂-, -C≡C- oder eine Einfachbindung,
n 0, 1 oder 2,
ein oder zwei nichtbenachbarte Reste Q N und die restlichen zweiwertigen Gruppen Q¹, Q² und Q³ C-Halogen bedeuten und, falls Q¹ und Q³ N bedeuten, Q² auch C-O(CF₂)ₛ-Y oder C-(CF₂)ₛ-Y bedeuten kann, worin
Y H, F oder Cl und
s 1 oder 2 bedeuten,
wobei die Verbindungen der Formel I mit L gleich H, n gleich 0, Q¹ und Q² gleich N und Q³ gleich CCl ausgenommen sind.

2. Heterocyclische Derivate nach Anspruch 1, gekennzeichnet durch die Formel

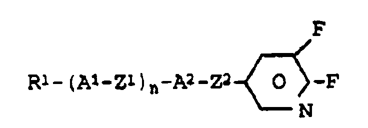

3. Heterocyclische Derivate nach Anspruch 1, gekennzeichnet durch die Formel

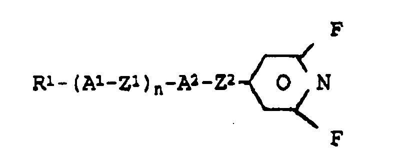

4. Heterocyclische Derivate nach Anspruch 1, gekennzeichnet durch die Formel

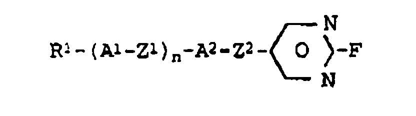

5. Heterocyclische Derivate nach Anspruch 1, gekennzeichnet durch die Formel

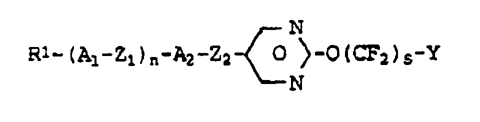

6. Heterocyclische Derivate nach Anspruch 1, gekennzeichnet durch die Formel

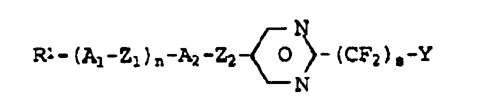

7. Heterocyclische Derivate nach den Ansprüchen 1 bis 6, wobei R¹ einen unverzweigten Alkylrest mit 2 bis 7 Kohlenstoffatomenbedeutet.

8. Heterocyclische Derivate nach den Ansprüchen 1 bis 7, wobei n 0 oder 1 bedeutet.

9. Heterocyclische Derivate nach den Ansprüchen 1 bis 8, wobei Z¹ und Z² jeweils eine Einfachbindung bedeuten.

10. Flüssigkristallines Medium, bei dem es sich um eine Mischung aus mindestens zwei Verbindungen handelt, dadurch gekennzeichnet, daß mindestens eine Verbindung ein heterocyclisches Derivat nach Anspruch 1 ist.

11. Flüssigkristall-Anzeigevorrichtung` dadurch gekennzeichnet, daß sie ein flüssigkristallines Medium nach Anspruch 10 enthält.

12. Elektrooptische Anzeigevorrichtung, dadurch gekennzeichnet, daß sie ein flüssigkristallines Medium nach Anspruch 10 enthält.

13. Verwendung von heterocyclischen Derivaten, gekennzeichnet durch die Formel 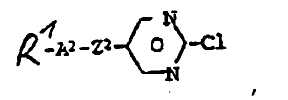 worin R¹, A² und Z² die in Anspruch 1 angegebene Bedeutung besitzen, in flüssigkristallinen Medien.

## Revendications

1. Dérivés hétérocycliques de formule I 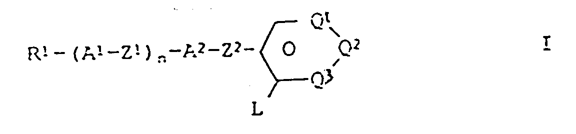 dans laquelle
R¹ désigne un groupe alkyle comportant jusqu'à 12 atomes de carbone dans lequel un ou deux groupes CH₂ non adjacents peuvent aussi être remplacés par -O-, -O-CO-, -CO-O-et/ou -CH=CH-,
L est H ou F,
A¹ et A², dans chaque cas, indépendamment l'un de l'autre, représentent un trans-1,4-cyclohexylène, dans lequel un ou deux groupes CH₂ non adjacents peuvent aussi être remplacés par -O- et/ou -S-, ou un 1,4-phénylène qui est non substitué ou substitué par un ou plusieurs atomes d'halogène et/ou groupes nitrile et/ou CH₃ et dans lequel un ou plusieurs groupes CH peuvent aussi être remplacés par N,
Z¹ et Z², dans chaque cas, indépendamment l' un de l'autre, représentent -CH₂CH₂-, -CO-O-, -O-CO-, -CH₂O-, -OCH₂-, -C≡C- ou une simple liaison,
n est 0, 1 ou 2,
un ou deux groupes Q non adjacents désignent N et les groupes divalents restants Q¹, Q² et Q³ sont C-halogène et,
dans le cas où Q¹ et Q³ sont N, Q² peut aussi être C-O(CF₂)ₛ-Y ou C-(CF₂)ₛ-Y, où
Y est H, F ou Cl, et
s est 1 ou 2,
à l'exception des composés de formule I, dans laquelle L est H, n est 0, Q¹ et Q² sont N et Q³ est CCl.

2. Dérivés hétérocycliques selon la revendication 1, caractérisés par la formule

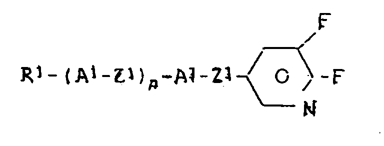

3. Dérivés hétérocycliques selon la revendication 1, caractérisés par la formule

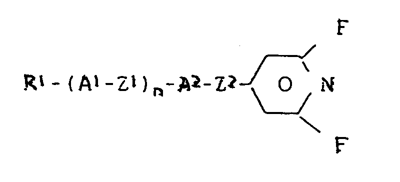

4. Dérivés hétérocycliques selon la revendication 1, caractérisés par la formule

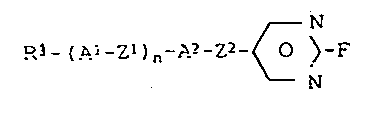

5. Dérivés hétérocycliques selon la revendication 1, caractérisés par la formule

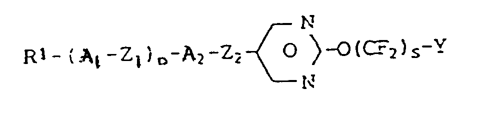

6. Dérivés hétérocycliques selon la revendication 1, caractérisés par la formule

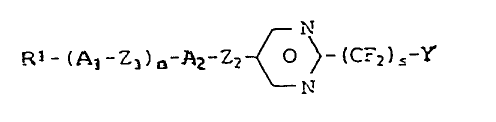

7. Dérivés hétérocycliques selon les revendications 1 à 6 dans lesquels R¹ désigne un radical alkyle non ramifié de 2 à 7 atomes de carbone.

8. Dérivés hétérocycliques selon les revendications 1 à 7, dans lesquels n est 0 ou 1.

9. Dérivés hétérocycliques selon les revendications 1 à 8, dans lesquels Z¹ et Z² sont chacun une simple liaison.

10. Milieu cristallin liquide qui est un mélange d'au moins deux composés, caractérisé en ce qu'au moins un composé est un dérivé hétérocyclique selon la revendication 1.

11. Dispositif d'affichage à cristaux liquides, caracterisé en ce qu'il contient un milieu cristallin liquide selon la revendication 10.

12. Dispositif d'affichage électro-optique, caractérisé en ce qu'il contient un milieu cristallin liquide selon la revendication 10.

13. Utilisation de dérivés hétérocycliques caractérisés par la formule 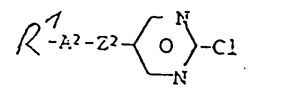 dans laquelle R¹, A² et Z² sont tels que définis dans la revendication 1, dans des milieux cristallins liquides.
